(19) Europäisches Patentamt
European Patent Office
Office européen des brevets

(11) Veröffentlichungsnummer: **0 226 636**
**B1**

(12) # EUROPÄISCHE PATENTSCHRIFT

(45) Veröffentlichungstag der Patentschrift: **08.02.89**

(51) Int. Cl.4: **C 07 C 147/06**, C 07 C 143/68, C 07 C 149/273, C 07 C 147/14, C 07 C 143/79, C 07 C 103/24, C 07 C 69/65, A 61 K 31/49, A 61 K 31/215, A 61 K 31/275, A 61 K 31/255

(21) Anmeldenummer: **86904797.7**

(22) Anmeldetag: **10.07.86**

(86) Internationale Anmeldenummer: **PCT/EP 86/00406**

(87) Internationale Veröffentlichungsnummer: **WO 87/00521 (29.01.87** Gazette **87/3)**

(54) **NEUE CARBONSÄUREDERIVATE, VERFAHREN ZU IHRER HERSTELLUNG, IHRE VERWENDUNG SOWIE ARZNEIMITTEL, DIE DIESE VERBINDUNGEN ENTHALTEN.**

(30) Priorität: **16.07.85 DE 3525284**

(43) Veröffentlichungstag der Anmeldung: **01.07.87 Patentblatt 87/27**

(45) Bekanntmachung des Hinweises auf die Patenterteilung: **08.02.89 Patentblatt 89/6**

(84) Benannte Vertragsstaaten: **AT BE CH DE FR GB IT LI LU NL SE**

(56) Entgegenhaltungen:
**GB-A- 1 382 267**

**Journal of the American Chemical Society, Volume 105, 1983, Easton, Pa. (US) B.M. Trost et al.: "Tungsten-catalyzed allylic alkylations. New avenues for Selectivity", pages 7757-7759, see page 7759, left hand column (cited in the application)**
**Journal of Organic Chemistry, volume 43, 1978, Easton, Pa. (US) B.M. Trost et al.: "Synthesis of thermodynamically less stable enol thioethers: An Alternative oxidative decarboxylation of alpha-thioacids", pages 4549-4551, see page 4549 (cited in the application)**

(73) Patentinhaber: **BOEHRINGER MANNHEIM GMBH, Sandhofer Strasse 116, D-6800 Mannheim 31 (DE)**

(72) Erfinder: **WOLFF, Hans, Peter, Rebenweg 24, D-6945 Hirschberg-Grossachsen (DE)**
Erfinder: **WITTE, Ernst-Christian, Beethovenstrasse 2, D-6800 Mannheim 1 (DE)**
Erfinder: **KÜHNLE, Hans-Frieder, Silcher Weg 6, D-6940 Weinheim (DE)**

**EP 0 226 636 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der vorliegenden Erfindung sind neue Carbonsäurederivate der allgemeinen Formel I,

$$\begin{array}{c} R_2 \\ | \\ R_1-A-C-COOH, \\ | \\ X \end{array} \qquad (I)$$

in welcher

$R_1$ einen gegebenfalls substituierten Aryl- oder Aryloxyrest,

$R_2$ Wasserstoff, einen niederen Alkylrest oder die Gruppe $R_1-A-$, sowie für den Fall, dass X die Cyanogruppe bedeutet, eine Acylamino- oder Aminogruppe,

A eine geradkettige oder verzweigte, gesättigte oder ungesättigte Alkylengruppe mit 3-8 Kohlenstoffatomen, die eine Kettenlänge von mindestens 3 Kohlenstoffatomen besitzt,

X die Cyanogruppe oder eine Gruppe der Formel $-B-R_3$ oder $-D-NR_4R_5$,

in welcher

B O, S, SO, $SO_2$, O(CO), $OSO_2$, NHCO, $NHSO_2$ oder CO,

D einen Valenzstrich, $SO_2$ oder CO,

$R_3$ eine Alkyl-, Trifluormethyl-, Cycloalkyl-, Aralkyl-, Aralkenyl- oder Arylgruppe,

wobei der jeweilige Arylrest gegebenenfalls substituiert sein kann,

$R_4$ Wasserstoff, einen niederen Alkylrest, einen gegebenenfalls substituierten Aryl- oder Aralkylrest,

$R_5$ Wasserstoff, einen niederen Alkylrest oder

$R_4$ und $R_5$ gemeinsam eine Alkylenkette mit 4-6 Kohlenstoffatomen, die durch O, S oder $NR_6$ unterbrochen sein kann, und

$R_6$ Wasserstoff, einen niederen Alkylrest oder einen gegebenenfalls substituierten Phenyl- oder Benzylrest bedeutet,

sowie deren physiologisch unbedenklichen Salze, Ester, Amide und Nitrile,

mit der Massgabe, dass für den Fall,

a) dass A eine Alkylengruppe mit 3 Kohlenstoffatomen ist,

$a_1$) in allen Fällen der Arylrest der Gruppe $R_1$, nicht den unsubstituierten Phenylrest und

$a_2$) X nicht die Gruppe $-CN$, $-NHCOR_3$ und $NR_4R_5$,

b) dass X ein Rest der Formel $-SCH_3$ ist, $R_2$ nicht Methyl,

c) dass X die Gruppe $-NH_2$ oder $-NHCOCH_3$ ist, $R_1A-$ nicht 4-Phenylbutyl oder 4-(4-Methoxyphenyl)butyl,

d) dass X die Gruppe 2,4-Dinitrophenyl ist, $R_1A-$ nicht 4-Phenylbutyl oder 5-Phenylpentyl,

e) dass X die Gruppe $-COCH_3$ ist, $R_1A-$ nicht 3-(2-Chlorphenyl)propyl oder 5-(4-Methoxyphenyl)pentyl

bedeuten darf.

Von den Verbindungen der allgemeinen Formel I sind mit Ausnahme der Norvalin-Analoga ($A = -(CH_2)_3-$ und $X = NR_4R_5$ bzw. $NHCOR_3$) bisher nur wenige Beispiele bekannt. Die erfindungsgemässe pharmakologische Wirkung ist hierbei noch nicht beschrieben worden:

2-Cyano-5-phenylpentansäure sowie Derivate mit Substituenten am aromatischen Ring werden als Reaktionsprodukte beschrieben. (Pallaud e.a., C.R. Acad. Sci., Ser. C *1971*, 273, 711)

2-Cyano-7-phenylheptansäure ist ebenfalls als Reaktionsprodukt beschrieben. (Julia e.a., Bull. Soc. Chim. Fr. *1968*, 3691; Chottard e.a., Compt. Rend. 1964, *259*, 2653)

2-Phenoxy-5-phenylpentansäure wird von Nordin, US-Patent 3.562.330, als Vorstufe von antiarrhythmisch wirksamen Aminen beschrieben.

5-Phenyl-2-phenylsulfonyl-4-pentansäuremethylester wird von Trost und Hung, J. Am. Chem. Soc. 1983, *105*, 7757, als Zwischenprodukt beschrieben.

2-Methyl-2-methylthio-5-phenylpentansäure und phenylsubstituierte Analoga sowie

2-Methyl-2-methylthio-6-(3-methoxyphenyl)hexansäure, und

2-Methyl-2-methylthio-7-(3,4-methylendioxiphenyl)heptansäure werden von Trost e.a., J. Org. Chem., *1978*, *43*, 4549, als Zwischenprodukte zur Herstellung von Enolthioethern verwendet.

Neben den bereits erwähnten Norvalinanaloga ($A = (-CH_2)_3-$) sind von den Derivaten mit Aminofunktion noch die folgenden beschrieben:

a) 2-Amino-6-phenylhexansäure und das N-Acetylderivat durch Pattabiraman e.a., Biochem. J., *1972*, *126*, 645, durch Kosui e.a., Mem. Fac. Sci., Kyushu Univ., Ser. C *1981*, *13*, 89 sowie durch Hashimoto e.a., Int. J. Pept. Protein Res., *1983*, *21*, 11.

b) 2-Amino-6-(4-methoxyphenyl)hexansäure und das N-Acetylderivat

durch Kosui e.a., Bull. Chem. Soc. Jpn., *1982*, *55*, 918 sowie

durch Mihara e.a., Int. J. Pept. Protein Res., *1984*, *23*, 447.

c) 2-[(2,4-Dinitrophenyl)amino]-6-phenylhexansäure und 2-[(2,4-Dinitrophenyl)amino]-7-phenylheptansäure

durch Kawai e.a., Tetrahedron Lett., *1975*, 2845 und Tetrahedron *1978*, *34*, 3435.

d) 2-Arylsulfonylamino-5-phenylpentansäuren:

durch Vieweg und Wagner in Pharmazie *1983*, *38*, 22 mit 4-Cyanophenyl als Arylrest sowie

durch Vieweg und Wagner in Pharmazie *1983*, *38*, 818 mit 4-Methylphenyl und 1-Naphthyl als Arylrest.

Die beiden letztgenannten Substanzen sind Zwischenprodukte für die Herstellung potentieller Serinproteinase-Hemmer.

e) 2-Acetyl-5-phenylpentansäure wird von Ansell e.a., J. Chem. Soc. Perkin Trans. *1*, *1973*, 2789, und Kaye e.a., Dos 2.060.443 sowie

2-Acetyl-5-(2-Chlorphenyl)pentansäure vom Amschler e.a., Eur. Pat. Appl. 25.192, beschrieben. Letztere dient zur Herstellung von antidiabetisch wirksamen Substanzen. Dem Zwischenprodukt selbst wird jedoch keine pharmakologische Wirkung zugeschrieben.

f) 2-Acetyl-7-(4-methoxyphenyl)heptansäure wird von Atkinson und Green, J. Chem. Soc., Perkin Trans. *1, 1974*, 394, als Reaktionsprodukt beschrieben.

g) 2-Acetoxy-5-phenylpentansäureethylester wird von Chottard e.a., Tetrahedron *1969, 25,* 4967, sowie

2-Aminocarbonyl-5-phenylpentansäure von Gardner und Brandon, J. Ord. Chem. *1957, 22,* 1704, als Reaktionsprodukt beschrieben.

Die Verbindungen der allgemeinen Formel I besitzen wertvolle pharmakologische Eigenschaften. Sie sind als Arzneimittel zur Behandlung von Diabetes, Prädiabetes und insbesondere zur Behandlung von Altersdiabetes geeignet.

Die Verbindungen der Formel I haben zu bekannten Antidiabetica strukturell und in der Wirkungsart keine Beziehung. Sie senken den Blutzuckerspiegel durch Verstärkung der peripheren Glucoseoxidation. Ihre Wirkung beruht auf einer Erhöhung der Sensitivität von peripherem Gewebe gegenüber Insulin. Im Gegensatz zu den Biguaniden wird dabei kein Anstieg der Blutlaktatwerte beobachtet. Die Verbindungen der allgemeinen Formel I stellen daher auch eine wertwolle Bereicherung bei der Behandlung nichtdiabetischer Krankheitszustände dar, bei denen eine Insulinresistenz vorhanden ist, wie z.B. Adipositas und Atherosklerose.

Es wurde weiterhin gefunden, dass die antidiabetische Wirksamkeit nicht auf die neuen Verbindungen der allgemeinen Formel I mit den vorgenannten Bedeutungen für $R_1$, $R_2$, A und X beschränkt ist, sondern dass auch bereits bekannte Derivate, die von der allgemeinen Formel I' eingeschlossen werden, mit der folgenden, erweiterten Bedeutung von $R_1'$, $R_2'$, A' und X' diese neu gefundene Wirksamkeit besitzen:

Gegenstand der Anmeldung sind daher auch Arzneimittel zur Behandlung von Diabetes, Prädiabetes, Adipositas und Atherosklerose, welche Carbonsäurederivate der allgemeinen Formel I' enthalten,

$$R_1'-A'-\underset{\displaystyle X'}{\overset{\displaystyle R_2'}{\overset{|}{\underset{|}{C}}}}-COOH \qquad (I')$$

in welcher

$R_1'$ Wasserstoff oder einen gegebenenfalls substituierten Aryl- oder Aryloxyrest,

$R_2'$ Wasserstoff, einen niederen Alkylrest oder die Gruppe $R_1-A'-$, sowie für den Fall, dass X die Cyanogruppe bedeutet, eine Acylamino- oder Aminogruppe,

A' eine geradkettige oder verzweigte, gesättigte oder ungesättigte Alkylengruppe mit 1-18 Kohlenstoffatomen,

X' die Cyano- oder Carbethoxygruppe oder eine Gruppe der Formel $-B-R_3$ oder $-D-NR_4R_5$, in welcher

B O, S, SO, $SO_2$, O(CO), $OSO_2$, NHCO, $NHSO_2$ oder CO und

D einen Valenzstrich, $SO_2$ oder CO,

$R_3$ eine Alkyl-, Trifluormethyl-, Cycloalkyl-, Aralkyl-, aralkenyl- oder Arylgruppe, deren Arylrest jeweils substituiert sein kann,

$R_4$ Wasserstoff, einen niederen Alkylrest, einen gegebenenfalls substituierten Aryl- oder Aralkylrest,

$R_5$ Wasserstoff, einen niederen Alkylrest oder

$R_4$ und $R_5$ gemeinsam eine Alkylenkette mit 4-6 Kohlenstoffatomen, die durch O, S oder $NR_6$ unterbrochen sein kann, und

$R_6$ Wasserstoff, einen niederen Alkylrest oder einen gegebenenfalls substituierten Phenyl- oder Benzylrest bedeutet,

sowie deren physiologisch unbedenklichen Salze, Ester, Amide und Nitrile.

Unter Arylresten werden aromatische Kohlenwasserstoffe mit 6 bis 14 Kohlenstoffatomen verstanden, bevorzugt die Phenyl- und Naphthylgruppe.

Unter substituierten Arylresten werden in allen Definitionen solche aromatische Kohlenwasserstoffe mit 6 bis 14 Kohlenstoffatomen verstanden, die in einer oder mehreren Positionen die Hydroxylgruppe, Halogen, niedere Alkyl-, niedere Alkoxy-, Trifluormethyl-, Cyano-, Nitro- oder gegebenenfalls ein- oder zweifach durch niedere Alkyl substituierte Aminogruppe tragen. Insbesondere kommen dafür gegebenenfalls durch die vorgenannten Gruppen substituierte Phenyl- oder Naphthylreste infrage. Besonders bevorzugt ist der Phenyl- und der 4-Chlorphenylrest. Dabei versteht man unter Halogen, Fluor, Chlor, Brom und Jod, vorzugsweise Fluor, Chlor und Brom.

Der Aryloxyrest enthält aromatische Kohlenwasserstoffe mit 6 bis 14 Kohlenstoffatomen, bevorzugt den Phenylrest. Unter substituierten Aryloxyresten sind solche zu verstehen, die in gleicher Weise wie die vorgenannten Arylreste substituiert sind, bevorzugt ist der 4-Chlorphenoxyrest.

Unter unverzweigten Alkylenketten A seien bevorzugt die folgenden verstanden:

$-(CH_2)_n-$, n in Formel I = 3-8, $-CH=CHCH_2-$, n in Formel I' = 1-18, $-C{\equiv}CCH_2-$.

Als verzweigte Gruppen A sind insbesondere bevorzugt die Gruppen

$$-CH_2\underset{\displaystyle CH_3}{\underset{|}{C}}HCH_2- \qquad \text{und} \qquad -CH=\underset{\displaystyle CH_3}{\underset{|}{C}}CH_2-$$

zu verstehen.

Unter den Alkylgruppen der Reste $R_3$ sind geradkettige oder verzweigte Kohlenwasserstoffe mit 1-8 Kohlenstoffatomen zu verstehen, insbesondere die Methyl- und die Octylgruppe.

Die Cycloalkylreste der Gruppe $R_3$ können 5 bis 7 Kohlenstoffatome im Ringsystem enthalten. Bevorzugt ist der Cyclohexylrest.

Unter Aralkyl- und Aralkenylresten der Gruppe $R_3$ sollen gesättigte oder ungesättigte Alkylreste mit 1-4 Kohlenstoffatomen verstanden werden, die durch eine gegebenenfalls substituierte Arylgruppe gemäss oben genannter Definition substituiert sind. Bevorzugt sind darunter der 2-Phenylethylrest und der 2-Phenylethenylrest zu verstehen.

Unter den niederen Alkylresten der Gruppen $R_4$, $R_5$ und $R_6$ sind Kohlenwasserstoffreste mit 1 bis

4 Kohlenstoffatomen zu verstehen, insbesondere der Methyl- und Ethylrest.

Unter Aralkylresten der Gruppen $R_4$ und $R_6$ sollen Alkylreste mit 1-3 Kohlenstoffatomen verstanden werden, die durch eine gegebenenfalls substituierte Arylgruppe gemäss oben genannter Definition substituiert sind. Bevorzugt ist darunter der Benzylrest zu verstehen.

Unter einer Gruppe $NR_4R_5$, bei der die Reste $R_4$ und $R_5$ gemeinsam eine Alkylenkette bilden, die durch O, S oder $NR_6$ unterbrochen sein kann, sind gesättigte Stickstoff-Heterocyclen mit 5-6 Ringatomen zu verstehen, die in beliebigen Positionen zusätzlich ein Sauerstoff-, Schwefel- oder Stickstoffatom enthalten können. Bevorzugt sind darunter die Piperidino-, Morpholino-, 5-Methylpiperazino-, 5-Phenylpiperazino- und 5-Benzylpiperazinogruppe zu verstehen.

Unter einer Gruppe der Formel $-B-R_3$ bzw. $-D-NR_4R_5$ sind bevorzugt die folgenden zu verstehen:

$-CN$, $-COOC_2H_5$,
$-OCH_3$, $-S-CH_3$, $-SOCH_3$, $-SO_2CH_3$, $-SO_2CF_3$,
$-O$-Phenyl, $-S$-Phenyl, $-SO$-Phenyl, $-SO_2$-Phenyl,
$-SO_2$-Naphthyl, $-SO_2CH_2CH_2$-Phenyl, $-SO_2-CH=CH$-Phenyl,
$-OCOCH_3$, $-OSO_2CH_3$, $-NHCOCH_3$, $-NHSO_2CH_3$, $-COCH_3$,
$-OCO$-Phenyl, $-OSO_2$-Phenyl, $-NHCO$-Phenyl, $-NHSO_2$-Phenyl, $-CO$-Phenyl,
$-NH_2$, $-N(C_2H_5)_2$, $-NH$-Phenyl, $-NH$-Benzyl,
-Morpholino, -Piperidino, -4-Benzylpiperazino,
$-SO_2NH_2$, $-SO_2N(C_2H_5)_2$, $-SO_2$-Piperidino,
$-CONH_2$, $-CON(CH_3)_2$, $-CO-N(C_2H_5)_2$, $-CO-NH$-Phenyl, $-CO-NH$-Benzyl,
$-CO$-Piperidino, $-CO$-Morpholino, $-CO$-(4-Phenylpiperazino),
$-CO$-(4-Benzylpiperazino),

wobei der Phenylring in allen Fällen durch die oben genannten Substituenten substituiert sein kann.

Für den Fachmann versteht es sich von selbst, dass im Falle einer ungesättigten Alkylenkette A ein eventuell vorhandener Aryloxysubstituent $R_1$ durch mindestens 1 gesättigtes Kohlenstoffatom von der Doppelbindung getrennt sein muss, da andernfalls unter der Gruppe $R_1-A-$ reaktive Enolether zu verstehen wären.

Als physiologisch unbedenkliche Salze kommen insbesondere Alkali-, Erdalkali- oder Ammoniumsalze (sowie gegebenenfalls Salze mit blutzuckersenkenden Biguaniden) in Frage.

Die von den Carbonsäuren der allgemeinen Formel I abgeleiteten Ester enthalten als Alkoholkomponente niedere einwertige Alkohole, von denen Methanol, Ethanol und n-Butanol bevorzugt sind, sowie mehrwertige Alkohole wie z.B. Glycerin, oder Alkohole mit anderen funktionellen Gruppen, wie z.B. Ethanolamin.

Die erfindungsgemässen, von den Carbonsäuren der allgemeinen Formel I abgeleiteten Amide enthalten als Aminkomponente vorzugsweise Ammoniak, p-Aminobenzoesäure, β-Alanin, Ethanolamin oder 2-Aminopropanol. Es kommen aber auch Alkylamine wie z.B. Isopropylamin oder tert.-Butylamin, Dialkylamine wie Diethylamin sowie cyclische Amine wie z.B. Morpholin oder 4-substituierte Piperazine in Frage.

Die substituierten Carbonsäuren der allgemeinen Formel I besitzen ein Chiralitätszentrum. Die obige Definition der erfindungsgemässen Verbindungen schliesst daher auch alle möglichen Enantiomeren, ihre Gemische und die Racemate ein.

Gegenstand der Erfindung sind weiterhin Verfahren zur Herstellung der Verbindungen der allgemeinen Formel I, indem man

A) in an sich bekannter Weise eine Verbindung der allgemeinen Formel II,

$$\begin{array}{c} Z_1 \\ | \\ H-C-W \\ | \\ Z_2 \end{array} \qquad (II)$$

in welcher

$Z_1$ $-COOR_7$, $-CN$, $-CO-R_3$, $-SO-R_3$, $-SO_2-R_3$, $-CONH_2$ oder $-SO_2NH_2$,

$Z_2$ Wasserstoff, niederes Alkyl oder $-NH-R_8$,

W $-COOR_7$ oder eine andere in die Carboxylfunktion überführbare Gruppe,

$R_7$ niederes Alkyl und

$R_8$ eine Aminoschutzgruppe

bedeuten und $R_2$ die oben angegebenen Bedeutung hat, entweder

a 1) mit einer Verbindung der allgemeinen Formel III,

$$R_1-A-Y \qquad (III)$$

in welcher $R_1$ und A die oben angegebenen Bedeutungen haben und Y einen reaktiven Rest darstellt, alkyliert oder

a 2) für den Fall, dass $Z_2$ Wasserstoff bedeutet, mit einer Verbindung der allgemeinen Formel III'

$$R_1-A'-CHO \qquad (III')$$

in welcher A' einen um die $CH_2$-Gruppe verkürzten Alkylenrest A bedeutet und $R_1$ die oben angegebene Bedeutung besitzt, kondensiert und im Anschluss an die Kondensation die entstandene Doppelbindung hydriert und

b) die aus den vorgenannten Verfahrensschritten erhaltenen Verbindungen der allgemeinen Formel IV,

$$\begin{array}{c} Z_1 \\ | \\ R_1-A-C-W \\ | \\ Z_2 \end{array} \qquad (IV)$$

gegebenenfalls umwandelt, indem man z.B.

b1) für den Fall, dass $Z_2$ Wasserstoff bedeutet, mit einer Verbindung der allgemeinen Formel V,

$$R_2-Y \qquad (V)$$

in welcher $R_2$ die oben genannte Bedeutung besitzt und Y eine reaktive Gruppe darstellt, erneut alkyliert oder

b2) für den Fall, dass $Z_1$ $-COOR_7$ und $Z_2$ die Gruppe $-NH-R_8$ bedeuten,
den Rest $R_8$ nach an sich bekannten Methoden in die Reste $-CO-R_3$ oder $-SO_2-R_3$ überführt oder

b3) für den Fall, dass $Z_1$ $COOR_7$ und $Z_2$ Wasserstoff bedeuten,
das Wasserstoffatom in an sich bekannter Weise mit einem Halogenierungsmittel durch ein Halogenatom austauscht, im Anschluss daran den Rest $Z_1$ durch Decarboxylierung in ein Wasserstoffatom überführt und das reaktive Derivat IV,
in welchem nun für $Z_2$ Halogen und für $Z_1$ Wasserstoff steht,
entweder mit einer Verbindung der Formel VI,

$$H-B-R_3 \qquad (VI)$$

in welcher B für O, S oder $NHSO_2$ steht und $R_3$ die oben angegebene Bedeutung hat,
zu der Verbindung

$$\begin{array}{c} H \\ | \\ R_1-A-C-W \\ | \\ B-R_3 \end{array} \qquad (IV')$$

oder mit einer Verbindung der Formel VI',

$$HNR_4R_5 \qquad (VI')$$

in welcher $R_4$ und $R_5$ die oben angegebene Bedeutung hat, zu der Verbindung

$$\begin{array}{c} H \\ | \\ R_1-A-C-W \\ | \\ NR_4R_5 \end{array} \qquad (IV'')$$

umsetzt und

c) im Anschluss an die Kondensation für den Fall, dass

c1) B ein Schwefelatom bedeutet, nach an sich bekannten Methoden gegebenenfalls zu den Sulfoxiden und Sulfonen oxidiert oder

c2) indem man das reaktive Derivat IV, worin $Z_1$ Wasserstoff und $Z_2$ Halogen bedeutet, mit Natriumsulfit umsetzt und die erhaltene Verbindung IV, in welcher $Z_2$ nun $-SO_3H$ bedeutet, in an sich bekannter Weise in die Gruppe $-SO_2NR_4R_5$ umwandelt und im Anschluss an diese gegebenenfalls durchgeführten Umwandlungen der Verbindungen IV die Gruppen W und gegebenenfalls $Z_1$, in die freien Carbonsäuren, ihre Salze, Ester oder Amide überführt.

B) Ein weiteres Verfahren zur Herstellung der Verbindungen der allgemeinen Formel I besteht darin, dass man eine Verbindung der allgemeinen Formel IV, in welcher $Z_1$ und $Z_2$ gemeinsam eine Oxofunktion darstellen, zunächst mit einem Reduktionsmittel oder einer metallorganischen Verbindung der allgemeinen Formel VII,

$$R_2-M \qquad (VII)$$

in welcher $R_2$ die oben angegebene Bedeutung hat und M ein Alkali- oder Erdalkalimetall bedeutet, umsetzt und die erhaltene Verbindung IV, in welcher $Z_1$ nun einen Rest der Formel $R_2$ und $Z_2$ eine Hydroxylgruppe bedeutet, in an sich bekannter Weise mit einem Sulfonylchlorid der allgemeinen Formel VIII,

$$R_3-SO_2Cl \qquad (VIII)$$

oder einem Carbonsäurechlorid der allgemeinen Formel IX,

$$R_3-COCl \qquad (IX)$$

in welcher $R_3$ die oben genannte Bedeutung hat, acyliert und im Anschluss daran gegebenenfalls die Gruppe W in die freie Carbonsäure, ihre Salze, Ester oder Amide überführt.

Bei Alkylierungen gemäss Verfahren a1) verwendet man als reaktive Derivate III entweder die Halogenide, insbesondere die Chloride und Bromide, oder aber geeignete Sulfonsäureester, z.B. Mesylate oder Tosylate.

Die Umsetzung der Halogenide bzw. Sulfonsäureester mit den Verbindungen der allgemeinen Formel II erfolgt zweckmässig unter Zusatz einer starken Base wie z.B. Natriumethylat, Natriumhydrid oder 1,8-Diazabicyclo-(5,4,0)undec-7-en. Als inertes Lösungsmittel dienen z.B. Ethanol, Dimethylsulfoxid oder Benzol. Weiterhin kommen z.B. Dimethylformamid oder Hexamethylphosphorsäuretriamid als Lösungsmittel in Betracht. Die Reaktion wird vorzugsweise bei Zimmertemperatur oder mässig erhöhter Temperatur oder bei der Siedetemperatur des verwendeten Lösungsmittels durchgeführt.

Die Umsetzung der Verbindungen II mit Aldehyden der Formel III' nach Verfahren a2) findet unter Bedingungen statt, wie sie für eine Kondensation aktivierter Methylengruppen mit Ketoverbindungen üblich sind. Vorzugsweise wird die Kondensation in Pyridin oder Dimethylformamid unter Zusatz katalytischer Mengen einer starken Base wie z.B. Piperidin durchgeführt.

Vorteilhaft setzt man dem Reaktionsgemisch ein geeignetes Lösungsmittel wie z.B. Benzol zu, um das Reaktionswasser azeotrop abzudestillieren.

Die nachfolgende Hydrierung der entstandenen Doppelbindung wird in üblicher Weise mit katalytisch erregtem Wasserstoff bei Normaldruck oder bei erhöhtem Druck durchgeführt. Als Katalysatoren kommen Metallkatalysatoren wie z.B. Raney-Nickel oder Palladium-Kohle in Betracht. Als Lösungsmittel sind z.B. Essigsäure oder niedere Alkohole, im Falle von Carbonsäure IV auch wässeriges Alkali geeignet.

Unter einer Aminoschutzgruppe $R_5$ sind z.B. Acylgruppen wie die Formyl- oder Acetylgruppe zu verstehen. Es kommen aber auch andere, z.B. die Peptidsynthesen verwendende Schutzgruppen, in Frage. Zur Umwandlung einer Verbindung der allgemeinen Formel IV mit geschützter Amino-

gruppe ($Z_1$ = $-NH-R_5$) in ein Derivat IV, $Z_1$ = $-NH-SO_2-R_3$, wird zunächst die Gruppe $R_5$ in üblicher Weise abgespalten, z.B. im Falle von Acylgruppen durch Verseifen mit verdünnter Alkalilauge oder verdünnter Mineralsäure. Anschliessend wird die erhaltene freie Aminosäure, zweckmässig nach Schutz der Carboxylfunktion, z.B. durch Verestern, in üblicher Weise durch ein Sulfonylchlorid der allgemeinen Formel VIII acyliert. Die Reaktion kann im Falle aromatischer Sulfonylchloride auch nach „Schotten-Baumann" vorgenommen werden. Will man unter wasserfreien Bedingungen arbeiten, so wird vorzugsweise absolutes Pyridin angewandt. Man kann aber auch andere tert. Basen wie z.B. Dimethylanilin oder Triethylamin in einem inerten Lösungsmittel wie z.B. Methylenchlorid einsetzen. Anstelle der freien Aminosäuren kann man auch deren Salze verwenden.

Zur Halogenierung der Verbindungen der allgemeinen Formel IV mit $Z_2$ = H finden vor allem Sulfurylchlorid, N-Chlorsuccinimid und N-Bromsuccinimid Anwendung. Umsetzungen mit N-Chlor- bzw. N-Bromsuccinimid werden vorzugsweise in einem inerten Lösungsmittel wie z.B. Tetrahydrofuran bei Raumtemperatur oder mässig erhöhter Temperatur durchgeführt. Die Reaktion erfolgt zweckmässig unter Zusatz einer starken Base wie z.B. Natriumhydrid. Umsetzungen mit Sulfurylchlorid finden vorzugsweise ohne Lösungsmittel in der Wärme, bevorzugt bei 50-80° C, statt.

Die Decarboxylierung von Verbindungen der allgemeinen Formel IV, in welcher W und $Z_1$ die Gruppe $-COOR_4$ bedeuten, wird zweckmässig gleichzeitig mit der Verseifung der entsprechenden Ester nach an sich bekannten Verfahren durchgeführt. Vorzugsweise findet die Decarboxylierung in einem Gemisch aus 6N Salzsäure und Eisessig oder in Natronlauge in der Siedehitze statt.

Die Umsetzung der α-Halogencarbonsäuren der allgemeinen Formel IV, worin $Z_1$ Wasserstoff und $Z_2$ Halogen bedeutet, mit den Verbindungen der allgemeinen Formel VI erfolgt zweckmässig unter Zusatz eines säurebindenden Mittels wie z.B. Natriumhydrogencarbonat, Natriumcarbonat, Kaliumcarbonat, Natriumethylat oder Natriumhydrid. Vorzugsweise werden für die Umsetzung Ester der α-Halogensäuren verwendet. Als inertes Lösungsmittel gelangen z.B. Ether, Benzol, Tetrahydrofuran, Dioxan oder Methylenchlorid zum Einsatz. Bei Verwendung anorganischer Basen verwendet man als Reaktionsmedium auch z.B. Ethanol, Butanon-2, Dimethylformamid, Hexamethylphosphorsäuretriamid oder Acetonitril.

Die Reaktionen werden bei Zimmertemperatur oder in der Hitze, bevorzugt in der Siedehitze, durchgeführt.

Die Oxidation von Verbindungen der allgemeinen Formel IV', in welcher B ein Schwefelatom bedeutet, zu Sulfoxiden oder Sulfonen wird vorzugsweise mit Wasserstoffperoxid in polaren Lösungsmitteln wie Eisessig, einem Gemisch von Eisessig und Acetanhydrid oder Aceton durchgeführt. Besonders vorteilhaft hat sich die Oxidation mit Trifluorperessigsäure erwiesen. Als Lösungsmittel wird hierbei zweckmässig Trifluoressigsäure verwendet.

Die Umsetzung von Verbindungen der allgemeinen Formel IV, in welcher $Z_1$ Wasserstoff und $Z_2$ Halogen bedeutet, mit Natriumsulfit wird in an sich bekannter Weise durch Erhitzen der Komponenten in wässeriger Lösung durchgeführt. Die erhaltenen Sulfocarbonsäuren werden in üblicher Weise, z.B. durch Thionylchlorid, in die reaktiven Sulfochloride überführt und diese dann mit Ammoniak zu den gewünschten Amidosulfonylderivaten umsetzt.

Die Reduktion von Verbindungen der allgemeinen Formel IV, in welcher $Z_1$ und $Z_2$ zusammen eine Oxogruppe bedeuten, nach Verfahren B kann in üblicher Weise mit katalytisch aktiviertem Wasserstoff durchgeführt werden. Bevorzugt ist dabei eine Hydrierung bei Normaldruck oder bei erhöhtem Druck in Gegenwart von Metallkatalysatoren wie z.B. Palladium oder Raney-Nickel in Lösungsmitteln wie z.B. Essigsäure oder niederen Alkoholen.

Gegebenenfalls kann auch mit komplexen Metallhydriden reduziert werden. Bevorzugt wird Natriumborhydrid eingesetzt. In diesem Fall kann die Umsetzung in einem Alkohol, insbesondere in Methanol, oder in Dioxan oder in wässerig-alkalischem Milieu durchgeführt werden.

Die Umsetzung von Verbindungen der allgemeinen Formel IV, in welcher $Z_1$ und $Z_2$ zusammen eine Oxofunktion bedeuten, mit metallorganischen Verbindungen der Formel VII nach Verfahren B findet in inerten Lösungsmitteln wie z.B. Diethylether oder Tetrahydrofuran statt. Die nachfolgende Acylierung der entstandenen Verbindungen IV, in welchen $Z_1$ nun $R_2$ und $Z_2$ die Hydroxylgruppe bedeuten, mit einer Verbindung der allgemeinen Formel VIII wird nach Methoden durchgeführt, wie sie bereits oben zur Acylierung der Aminosäuren beschrieben sind. Bevorzugt findet die Reaktion in Pyridin als Lösungsmittel statt.

Unter der in eine Carboxylfunktion überführbaren Gruppe W soll insbesondere die Nitrilgruppe oder ein Rest verstanden werden, der sich oxidativ in die Carboxylfunktion überführen lässt. Als oxidierbare Gruppen kommen vorzugsweise die Hydroxymethyl-, die Aminomethyl- und die Formylgruppe oder funktionelle Derivate dieser Gruppen in Frage. Die Oxidation lässt sich mit den üblichen Oxidationsmitteln wie z.B. Mangan-IV-verbindungen, Permanganaten, Dichromaten, im Falle der Formylgruppe auch mit Luftsauerstoff und Silberoxid durchführen.

Die gegebenenfalls im Anschluss an die Kondensation zu den Verbindungen der allgemeinen Formel IV durchzuführende Umwandlung der Substituenten W und $Z_1$ erfolgt beispielsweise durch Verseifen der Carbonsäureester zu den entsprechenden Carbonsäuren mit Mineralsäuren oder Alkalihydroxiden in einem polaren Lösungsmittel (wie Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei mässig erhöhten Temperaturen durchgeführt. Umgekehrt kann man aber auch die Carbonsäuren in üblicher Weise verestern oder Ester mit einem bestimmten Rest $R_4$ durch Umestern in einen Ester mit einem anderen Rest $R_4$ umwandeln. Die Veresterung der Carbonsäuren wird zweckmässig in Gegenwart eines sauren Katalysators, wie z.B. Chlorwasserstoff, Schwefelsäure, p-Toluolsulfonsäure oder eines stark sauren Ionenaustauschharzes, vorgenommen.

Umesterungen hingegen erfordern den Zusatz einer geringen Menge einer basischen Substanz, z.B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol sowie mehrwertige Alkohole, z.B. Glycerin, oder Alkohole mit anderen funktionellen Gruppen, wie z.B. Ethanolamin.

Die erfindungsgemässen, von den Carbonsäuren der allgemeinen Formel I abgeleiteten Amide werden bevorzugt nach an sich bekannten Methoden aus den Carbonsäuren oder ihren reaktiven Derivaten (wie z.B. Carbonsäurehalogeniden, -estern, -aziden, -anhydriden oder gemischten Anhydriden) durch Umsetzung mit Aminen hergestellt. Als Aminkomponenten kommen z.B. Ammoniak, Alkylamine, Dialkylamine, aber auch Aminoalkohole wie z.B. Ethanolamin und 2-Aminopropanol sowie Aminosäuren wie z.B. p-Aminobenzoesäure, β-Alanin und andere in Frage. Andere wertvolle Aminkomponenten sind Alkyl-, Aralkyl- und Arylpiperazine.

Die Herstellung der obigen Amide kann aber auch durch partielle Verseifung der von den erfindungsgemässen Carbonsäuren abgeleiteten Nitrilen erfolgen. Die Verseifung erfolgt in verdünnten Mineralsäuren bei mässig erhöhten Temperaturen, in alkalischer Hydroperoxidlösung oder vorteilhaft in 98proz. Schwefelsäure oder Polyphosphorsäure.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglutamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süssstoffe enthalten.

Die verabreichte Dosierung hängt vom Alter, der Gesundheit und dem Gewicht des Empfängers, dem Ausmass der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg/Tag in einer oder mehreren Anwendungen pro Tag wirksam, um die gewünschten Resultate zu erhalten.

Bevorzugt im Sinne der Erfindung sind ausser den in den Beispielen genannten Verbindungen der Formel I sowie deren Ester und Amiden die folgenden:

2-[(4-Methylphenyl)sulfonyl]-8-phenyloctansäure

2-[(4-Methylphenyl)sulfonyl]-5-phenyl-4-pentinsäure

6-(4-Methylphenyl)-2-[(4-methylphenyl)sulfonyl]hexansäure

7-(4-Methylphenyl)-2-[(4-methylphenyl)sulfonyl]heptansäure

8-(4-Methylphenyl)-2-[(4-methylphenyl)sulfonyl]octansäure

10-(4-Methylphenyl)-2-[(4-methylphenyl)-sulfonyl]decansäure

7-(4-Chlorphenyl)-2-(amidosulfonyl)heptansäure

7-(4-Chlorphenyl)-2-(phenoxy)heptansäure

7-(4-Chlorphenyl)-2-(4-methylphenoxy)heptansäure

7-(4-Chlorphenyl)-2-(phenylthio)heptansäure

7-(4-Chlorphenyl)-2-[(4-methylphenyl)thio]-heptansäure

7-(4-Chlorphenyl)-2-(phenylsulfinyl)heptansäure

7-(4-Chlorphenyl)-2-([4-methylphenyl)sulfinyl]heptansäure

6-(4-Chlorphenyl)-2-[(4-methylphenyl)sulfonyl]hexansäure

7-(4-Chlorphenyl)-2-(octylsulfonyl)heptansäure

7-(4-Chlorphenyl)-2-[(3-chlorphenyl)sulfonyl]heptansäure

7-(4-Chlorphenyl)-2-[(4-methoxyphenyl)sulfonyl]heptansäure

7-(4-Chlorphenyl)-2-[(3-trifluormethylphenyl)sulfonyl]heptansäure

7-(4-Chlorphenyl)-2-[(4-cyanophenyl)sulfonyl]heptansäure

7-(4-Chlorphenyl)-2-[(4-nitrophenyl)sulfonyl]heptansäure

7-(4-Chlorphenyl)-2-[(4-dimethylaminophenyl)sulfonyl]heptansäure

7-(4-Chlorphenyl)-2-[(4-hydroxyphenyl)sulfonyl]heptansäure

7-(4-Chlorphenyl)-2-[(4-acetylphenyl)sulfonyl]heptansäure

7-(4-Chlorphenyl)-2-[(2-phenylethyl)sulfonyl]heptansäure

7-(4-Chlorphenyl)-2-[(2-phenylethenyl)sulfonyl]heptansäure

7-(4-Chlorphenyl)-2-[(5-(4-chlorphenyl)-pentyl)sulfonyl]heptansäure

7-(4-Chlorphenyl)-2-(naphth-1-ylsulfonyl)-heptansäure

7-(4-Chlorphenyl)-2-(naphth-2-ylsulfonyl)-heptansäure

9-(4-Chlorphenyl)-2-[(4-methylphenyl)sulfonyl]nonansäure

10-(4-Chlorphenyl)-2-[(4-methylphenyl)sulfonyl]decansäure

7-(4-Chlorphenoxy)-2-[(4-methylphenyl)sulfonyl]heptansäure

7-(4-Methoxyphenyl)-2-(phenylsulfonyl)-heptansäure

7-(4-Methoxyphenyl)-2-[(4-methylphenyl)-sulfonyl]heptansäure

2-[(4-Chlorphenyl)sulfonyl]-7-(4-methoxyphenyl)heptansäure

7-(4-Methoxyphenyl)-2-[(4-methoxyphenyl)sulfonyl]heptansäure

2-(Phenylsulfonyl)-7-(3-trifluormethylphenyl)heptansäure

2-[(4-Methylphenyl)sulfonyl]-7-(3-trifluormethylphenyl)heptansäure

2-[(4-Chlorphenyl)sulfonyl]-7-(3-trifluormethylphenyl)heptansäure

2-[(4-Methoxyphenyl)sulfonyl]-7-(3-trifluormethylphenyl)heptansäure

7-(3,4-Dichlorphenyl)-2-(phenylsulfonyl)-heptansäure

7-(3,4-Dichlorphenyl)-2-[(4-methylphenyl)-sulfonyl]heptansäure

2-[(4-Chlorphenyl)sulfonyl]-7-(3,4-dichlorphenyl)heptansäure

7-(3,4-Dichlorphenyl)-2-[(4-methoxyphenyl)sulfonyl]heptansäure

2-[(4-Methylphenyl)sulfonyl]-7-(naphth-1-yl)heptansäure

2-[(4-Methylphenyl)sulfonyl]-7-(naphth-2-yl)heptansäure

*Beispiel 1*

*7-(4-Chlorphenyl)-2-[(4-methylphenyl)sulfonyl]heptansäure*

a) Zu einer Lösung von 40 mmol Natriumethylat in 55 ml absolutem Ethanol fügt man unter Rühren bei 50° C 9,69 g (40 mmol) [(4-Methylphenyl)sulfonyl]essigsäureethylester zu. Es entsteht eine Suspension, die man unter Erhitzen auf Rückflusstemperatur tropfenweise mit 10,46 g (30 mmol) 5-(4-Chlorphenyl)pentylbromid versetzt. Der Niederschlag geht dabei allmählich in Lösung. Man rührt das Reaktionsgemisch weiter bei Rückflusstemperatur, wobei allmählich neue Kristalle ausfallen. Nach 5 h dampft man ein, versetzt den Rückstand mit Eiswasser und extrahiert das Gemisch mehrfach mit Essigester.

Die vereinigten organischen Phasen werden über Natriumsulfat getrocknet, eingedampft und der Rückstand mit einem Gemisch von Chloroform und Toluol (2:1) an Kieselgel chromatographiert.

Ausbeute: 15,7 g (93% d.Th.) 7-(4-Chlorphenyl)-2-[(4-methylphenyl)sulfonyl]heptansäureethylester, farbloses Öl.

b) Man hält ein Gemisch aus 4,2 g (9,9 mmol) des obigen Ethylesters, 20 ml 1N Kalilauge und 125 ml Methanol 2 h auf 40° C und gibt dann 20 ml 1N Salzsäure zu. Anschliessend dampft man das Methanol im Vakuum ab und extrahiert den wässerigen Rückstand mehrfach mit Methylenchlorid. Die vereinigten organischen Extrakte werden über Natriumsulfat getrocknet und eingedampft. Ausbeute: 3,05 g (78% d.Th.) 7-(4-Chlorphenyl)-2-[(4-methylphenyl)sulfonyl]heptansäure, farbloses Öl.

Das Natriumsalz wird durch Umsetzung mit stöchiometrischen Mengen wässeriger Natriumhydrogencarbonat-Lösung und Eindampfen der erhaltenen Lösung hergestellt. Fp. 90-100° C (amorph).

In Analogie zu a) und b) erhält man aus [(4-Methylphenyl)sulfonyl]essigsäureethylester und

3-(4-Chlorphenyl)propylbromid:

a-1) 5-(4-Chlorphenyl)-2-[4-methylphenyl)-sulfonyl]heptansäureethylester, farbloses Öl, Ausb. 87% d.Th.

b-1) 5-(4-Chlorphenyl)-2-[(4-methylphenyl)-sulfonyl]heptansäure, Natriumsalz: Fp. 130° C (Zers.), Ausb. 63% d.Th.

4-Chlorcinnamylchlorid:

a-2) 5-(4-Chlorphenyl)-2-[(4-methylphenyl)-sulfonyl]-4-pentensäureethylester, Fp. 87-88° C, Ausb. 46% d.Th.

b-2) 5-(4-Chlorphenyl)-2-[(4-methylphenyl)-sulfonyl]-4-pentensäure, Fp. 162-164° C, Ausb. 82% d.Th.

4-(4-Chlorphenoxy)butylbromid:

a-3) 6-(4-Chlorphenoxy)-2-[(4-methylphenyl)sulfonyl]hexansäureethylester, farbloses Öl, Ausb. 67% d.Th.

b-3) 6-(4-Chlorphenoxy)-2-[(4-methylphenyl)sulfonyl]hexansäure, Natriumsalz: Fp. 168-171° C, Ausb. 82% d.Th.

6-(4-Chlorphenyl)-hexylbromid:

a-4) 8-(4-Chlorphenyl)-2-[(4-methylphenyl)-sulfonyl]octansäureethylester, farbloses Öl, Ausb. 76% d.Th.

b-4) 8-4(Chlorphenyl)-2-[(4-methylphenyl)-

sulfonyl]octansäure, farbloses Öl, Ausb. 70% d.Th.

5-Phenylpentylbromid:

a-5) 5-Phenyl-2-[(4-methylphenyl)sulfonyl]-heptansäureethylester, farbloses Öl, Ausb. 75% d.Th.

b-5) 5-Phenyl-2-[(4-methylphenyl)sulfonyl]-heptansäure, farbloses Öl, Ausb. 63% d.Th.

*Beispiel 2*

*7-(4-Chlorphenyl)-2-[(4-methylphenyl)sulfonyl-oxy]heptansäureethylester*

a) Man versetzt eine Lösung von 11,25 g (44 mmol) 7-(4-Chlorphenyl)-2-oxoheptansäure in 175 ml Ethanol bei 50° C unter Rühren rasch mit einer Lösung von 1,84 g (48,6 mmol) Natriumborhydrid in 175 ml Ethanol. Dann lässt man 3 h bei Zimmertemperatur stehen, dampft das Reaktionsgemisch ein und nimmt den Rückstand in Wasser auf. Nach dem Ansäuern wird die wässerige Phase mit Essigester extrahiert. Die organischen Extrakte werden getrocknet und eingedampft. Das zurückbleibende Öl wird in wässeriger Natriumhydrogencarbonat-Lösung gelöst, die Lösung mit Kohle geklärt und das nach dem Ansäuern abgeschiedene Öl in Ether aufgenommen. Durch Eindampfen der getrockneten Etherlösung erhält man 7,4 g (65% d.Th.) 7-(4-Chlorphenyl)-2-hydroxyheptansäure, Fp. 78-81° C.

b) Man erhitzt ein Gemisch aus 5,2 g (20,3 mmol) der obigen α-Hydroxycarbonsäure, 1 g Amberlite IR 120 (saure Form) und 50 ml absolutes Ethanol unter Rühren 25 h auf Rückflusstemperatur. Dann saugt man vom Ionenaustauscher-Harz ab und dampft das Filtrat ein. Ausbeute: 5,7 g (quant.) 7-(4-Chlorphenyl)-2-hydroxyheptansäureethylester, farbloses Öl.

c) Eine Lösung von 10,0 g (35,1 mmol) des obigen Ethylesters in 70 ml trockenem Pyridin werden unter Rühren bei 0° C mit 13,4 g (70,2 mmol) p-Toluolsulfonylchlorid innerhalb 40 min portionsweise versetzt. Anschliessend lässt man das Gemisch über Nacht kühl stehen, giesst es dann auf Eis und extrahiert die wässerige Phase mehrfach mit Essigester. Die vereinigten Essigester-Extrakte werden dreimal mit 1 N Salzsäure gewaschen, getrocknet und eingedampft. Ausbeute: 13,0 g (84% d.Th.) 7-(4-Chlorphenyl)-2-[(4-Methylphenyl)sulfonyl]heptansäureethylester, farbloses Öl.

In Analogie zu c) werden aus 7-(4-Chlorphenyl)-2-(hydroxy)heptansäureethylester die folgenden Produkte hergestellt:

c-1) mit Methansulfonylchlorid

7-(4-Chlorphenyl)-2-(methylsulfonyloxy)heptansäureethylester, farbloses Öl, Ausb. 80% d.Th.

c-2) mit Benzolsulfonylchlorid

7-(4-Chlorphenyl)-2-(phenylsulfonyloxy)heptansäureethylester, farbloses Öl, Ausb. 62% d.Th.

c-3) mit 4-Chlorbenzolsulfonylchlorid

7-(4-Chlorphenyl)-2-[(4-chlorphenyl)sulfonyl-oxy]heptansäureethylester, farbloses Öl, Ausb. 67% d.Th.

*Beispiel 3*

*7-(4-Chlorphenyl)-2-methyl-2-[(4-methylphenyl)sulfonyl]heptansäureethylester*

a) Zu einer Lösung von 8,3 g (29,3 mmol) 7-(4-Chlorphenyl)-2-oxoheptansäureethylester in 60 ml abs. Ether werden unter Rühren und Eiskühlung 29,3 mmol einer frisch bereiteten Lösung von Methylmagnesiumjodid in Ether tropfenweise zugefügt. Dann wird das Kühlbad entfernt und das Gemisch noch 2 h bei Raumtemperatur nachgerührt. Anschliessend zersetzt man mit Natriumchloridlösung, trennt die organische Phase ab, trocknet und dampft ein. Ausbeute: 7,05 g (80% d.Th.) 7-(4-Chlorphenyl)-2-hydroxy-2-methylheptansäureethylester, farbloses Öl.

b) Zu einer Lösung von 7,05 g (23,6 mmol) des obigen 2-Hydroxycarbonsäureethylesters in 100 ml trockenem Pyridin fügt man unter Rühren bei 0° C portionsweise 9,42 g (47,2 mmol) p-Toluolsulfonylchlorid hinzu und lässt das Reaktionsgemisch über Nacht in der Kälte stehen. Dann giesst man auf Eis, extrahiert mit Essigester und wäscht die vereinigten Extrakte dreimal mit 1 N Salzsäure. Anschliessend wird getrocknet und eingedampft. Den Rückstand chromatographiert man mit einem Gemisch von Toluol und Chloroform (1:1) an Kieselgel. Ausbeute: 6,0 g (54% d.Th.) 7-(4-Chlorphenyl)-2-methyl-2-[(4-methylphenyl)sulfonyl-oxy]heptansäureethylester, farbloses Öl.

*Beispiel 4*

*7-(4-Chlorphenyl)-2-[[(4-methylphenyl)sulfonyl]amino]heptansäure*

a) Man versetzt eine Lösung von 2,46 g (107 mmol) Natrium in 100 ml abs. Ethanol mit 17,6 g (103 mmol) Acetamidocyanessigsäureethylester und 25,5 g (97,5 mmol) 5-(4-Chlorphenyl)-pentylbromid und erhitzt das Gemisch unter Rühren 6 h auf Rückflusstemperatur. Anschliessend dampft man das Ethanol ab, nimmt den Rückstand in Wasser auf und stellt durch Zugabe von wenig verdünnter Salzsäure auf pH 5. Dann extrahiert man mit Essigester, trocknet die vereinigten Extrakte über Natriumsulfat und dampft sie ein. Der Rückstand wird in wenig Ether aufgenommen und in der Kälte zur Kristallisation gebracht. Ausbeute: 16,0 g (47% d.Th) 2-Acetamido-7-(4-chlorphenyl)-2-cyanoheptansäureethylester, Fp. 73-77° C.

b) Man erhitzt ein Gemisch von 16,0 g (45,6 mmol) des obigen Ethylesters, 16 Ätznatron und 160 ml Wasser 22 h unter Rühren auf Rückflusstemperatur. Dann wird mit 6N Salzsäure auf pH 6 gestellt und die ausgefallenen Kristalle in der Kälte abgesaugt. Ausbeute: 11,3 g (97% d.Th) 7-(4-Chlorphenyl)-2-aminoheptansäure, Fp. 218-220° C.

c) Ein Gemisch von 11,9 g (46,5 mmol) der obigen Aminosäure und 120 ml absolutes Ethanol werden unter Eiskühlung mit Chlorwasserstoff gesättigt und anschliessend über Nacht bei Raumtemperatur belassen. Das Reaktionsgemisch wird mit Kohle geklärt und die Lösung eingedampft.

Der Rückstand wird mit Ether behandelt und die entstandenen Kristalle in der Kälte abgesaugt. Ausbeute: 8,6 g (58% d.Th.) 7-(4-Chlorphenyl)-2-aminoheptansäureethylester-hydrochlorid, Fp. 101-102° C.

d) In eine Lösung von 4,35 g (13,6 mmol) des obigen Aminosäureesters in 45 ml trockenem Pyridin trägt man unter Rühren bei 0° C portionsweise 2,85 g (14,9 mmol) 4-Toluolsulfonsäurechlorid ein. Man rührt noch 3 h unter Eiskühlung nach, giesst das Reaktionsgemisch dann auf Eis und säuert mit conc. Salzsäure an. Man extrahiert die wässerige Phase mit Ether und wäscht die Etherextrakte zweimal mit 0,5N Salzsäure. Dann wird die Lösung getrocknet, mit Kohle entfärbt und eingedampft. Der Rückstand wird durch Verreiben unter Ligroin zur Kristallisation gebracht. Ausbeute: 3,6 g (60% d.Th.) 7-(4-Chlorphenyl)-2-[(3-methylphenyl)sulfonylamino]heptansäureethylester, Fp. 56-58° C.

e) Man rührt ein Gemisch aus 4,0 g (9,1 mmol) des obigen sulfonierten Aminoesters, 23 ml 1N Kalilauge und 55 ml Methanol 6 h bei 40° C. Dann klärt man die Lösung mit Kohle, säuert mit 23 ml 1N Salzsäure an, verdünnt mit Wasser und nimmt das abgeschiedene Öl in Essigester auf. Die Essigester-Lösung wird getrocknet und eingedampft. Der Rückstand wird unter Ligroin zur Kristallisation gebracht. Ausbeute: 2,6 g (70% d.Th.) 7-(4-Chlorphenyl)-2-[(4-methylphenyl)sulfonylamino]heptansäure, Fp. 88-90° C.

In Analogie zu d) und e) werden aus 7-(4-Chlorphenyl)-2-aminoheptansäureethylester-hydrochlorid und Methansulfonylchlorid die folgenden Produkte dargestellt:

d-1) 7-(4-Chlorphenyl)-2-[(methylsulfonyl)amino]heptansäureethylester, Fp. 60-62° C, Ausb. 68% d.Th.

e-1) 7-(4-Chlorphenyl)-2-[(methylsulfonyl)amino]heptansäure, Fp. 73-75° C, Ausb. 82% d.Th.

*Beispiel 5*

*7-(4-Chlorphenyl)-2-[(4-chlorphenyl)sulfonyl]heptansäureethylester*

Nach der in Beispiel 1a) beschriebenen Arbeitsweise erhält man aus 40 mmol Natriumethylat, 10,51 g (40 mmol) (4-Chlorphenyl)sulfonylessigsäureethylester und 10,46 g (40 mmol) 5-(4-Chlorphenyl)pentylbromid in 100 ml abs. Ethanol 9,3 g (52% d.Th.) 7-(4-Chlorphenyl)-2-[(4-chlorphenyl)sulfonyl] - heptansäureethylester, farbloses Öl.

In analoger Weise erhält man aus 5-(4-Chlorphenyl)pentylbromid und

a) *Trifluormethylsulfonylessigsäureethylester:*

5-(4-Chlorphenyl)-2-(Trifluormethylsulfonyl)-heptansäureethylester, farbloses Öl, Ausb. 56% d.Th.

b) *Methylsulfonylessigsäureethylester:*

5-(4-Chlorphenyl)-2-(methylsulfonyl)heptansäureethylester, Fp. 60-62° C, Ausb. 64% d.Th.

c) *Phenylsulfonylessigsäureethylester:*

5-(4-Chlorphenyl)-2-(phenylsulfonyl)heptansäureethylester, farbloses Öl, Ausb. 77% d.Th.

d) *2-[(4-Methylphenyl)sulfonyl]propionsäureethylester:*

5-(4-Chlorphenyl)-2-methyl-2-[(4-methylphenyl)sulfonyl]heptansäureethylester, farbloses Öl, Ausb. 74% d.Th.

*Beispiel 6*

*7-(4-Chlorphenyl)-2-[(4-chlorphenyl)sulfonyl]heptansäure*

Nach der in Beispiel 1b) beschriebene Arbeitsweise erhält man aus 6,9 g (15,6 mmol) 7-(4-Chlorphenyl)-2-[(4-chlorphenyl)sulfonyl]heptansäureethylester (Beispiel 5) und 34 ml 1N Kalilauge in 100 ml Methanol 5,1 g (75% d.Th.) des Natriumsalzes der 7-(4-Chlorphenyl)-2-[(4-chlorphenyl)sulfonyl]heptansäure, amorphes Pulver.

In analoger Weise erhält man aus

a) 7-(4-Chlorphenyl)-2-(methylsulfonyl)heptansäureethylester (Beispiel 5b):

7-(4-Chlorphenyl)-2-(methylsulfonyl)heptansäure, Fp. 81-84° C, Ausb. 85% d.Th.

b) 7-(4-Chlorphenyl)-2-(phenylsulfonyl)-heptansäureethylester (Beispiel 5c)

7-(4-Chlorphenyl)-2-(phenylsulfonyl)heptansäure, Fp. 95-97° C, Ausb. 71% d.Th.

c) 7-(4-Chlorphenyl)-2-methyl-2-[(4-methylphenyl)sulfonyl]heptansäureethylester (Beispiel 5d):

7-(4-Chlorphenyl)-2-methyl-2-[(4-methylphenyl)sulfonylheptansäure, farbloses Öl, Ausb. 62% d.Th.

*Beispiel 7*

*5-(4-Chlorphenyl)-2-[3-(4-chlorphenyl)-2-propenyl]-2-[(4-methylphenyl)sulfonyl]-4-pentansäureethylester*

Man versetzt eine Lösung von 40 mmol Natriumethylat in 100 ml abs. Ethanol bei 60° C unter Rühren mit 4,35 g (20 mmol) (4-Methylphenyl)-sulfonylessigsäureethylester. Dann lässt man 15 min ausreagieren und versetzt den entstandenen Niederschlag mit 7,48 g ( 40 mmol) 4-Chlorcinnamylchlorid. Das Reaktionsgemisch wird anschliessend 5 Stunden auf Rückflusstemperatur erhitzt, eingedampft und der Rückstand mit Ether behandelt. Die Etherlösung wird mit Wasser gewaschen, getrocknet und eingedampft. Den Rückstand chromatographiert man mit Toluol an Kieselgel. Man erhält 13,4 g (62% d.Th.) Titelverbindung, Fp. 75-78° C.

*Beispiel 8*

*7-(4-Chlorphenyl)-2-[(4-methylphenyl)sulfonyl]heptannitril*

Man rührt ein Gemisch aus 4,5 g (17,2 mmol) 5-(4-Chlorphenyl)pentylbromid, 3,36 g (17,2 mmol) (4-Methylphenyl)sulfonylacetonitril, 2,26 g (17,2 mmol) 1,8-Diazabicyclo(5,4,0)undec-7-en und 30 ml Benzol 6 Stunden bei Raumtemperatur. Dann wäscht man die Reaktionsmischung mit

Wasser, trocknet die organische Phase und engt sie ein. Den Rückstand chromatographiert man mit einem Gemisch von Toluol und Dioxan (10:1) an Kieselgel. Ausbeute: 4,2 g (65% d.Th.) der Titelverbindung, farbloses Öl.

*Beispiel 9*

*7-(4-Chlorphenyl)-2-(4-methylphenoxy)heptansäure*

a) Man versetzt eine Lösung von 63,4 g (0,186 mol) 7-(4-Chlorphenyl)-2-ethoxycarbonylheptansäureethylester (BM 13.861) in 500 ml abs. Tetrahydrofuran portionsweise mit 8,1 g (0,186 mol) 55proz. Natriumhydrid in Mineralölsuspension und rührt zur Vervollständigung der Salzbildung 15 min nach. Dann fügt man 33,1 g (0,186 mol) N-Bromsuccinimid zu und rührt das Gemisch 8 h bei Zimmertemperatur. Danach wird in Wasser gegossen, angesäuert und mit Ether extrahiert. Die vereinigten Extrakte werden gewaschen, getrocknet und eingedampft. Den Rückstand chromatographiert man mit Toluol an Kieselgel. Man erhält 61,3 g (78% d.Th.) 2-Brom-7-(4-chlorphenyl)-2-ethoxycarbonyl-heptansäureethylester, farbloses Öl.

b) Ein Gemisch von 102,7 g (0,245 mol) 2-Brom-7-(4-chlorphenyl)-2-ethoxycarbonyl-heptansäureethylester, 367 ml Essigsäure und 367 ml 6N Bromwasserstoffsäure werden 60 Stunden auf Rückflusstemperatur erhitzt. Dann wird mit Wasser verdünnt und mit Ether extrahiert. Die Extrakte werden mit Kohle geklärt, getrocknet und eingedampft.

Der Eindampfrückstand wird mit Ligroin verrieben. Man erhält 67,8 g (86% d.Th.) 2-Brom-7-(4-chlorphenyl)heptansäure, Fp. 77-79° C.

c) Zu einer Lösung von 31,0 g (0,097 mol) 2-Brom-7-(4-Chlorphenyl)heptansäure in 250 ml abs. Ether, der 0,5 ml Methanol enthält, wird unter Rühren bei Zimmertemperatur eine Lösung zugetropft, bis die Stickstoffentwicklung aufhört. Dann dampft man das Reaktionsgemisch ein und chromatographiert den Rückstand mit Toluol an Kieselgel. Man erhält 18,1 g (56% d.Th.) 2-Brom-7-(4-chlorphenyl)heptansäuremethylester, farbloses Öl.

d) Man erhitzt ein Gemisch von 5,6 g (17 mmol) 2-Brom-7-(4-chlorphenyl)heptansäuremethylester, 1,82 g (17 mmol) p-Kresol, 7,0 g (50 mmol) Kaliumcarbonat und 70 ml Butanon 36 h auf Rückflusstemperatur. Dann saugt man den anorganischen Niederschlag ab und dampft das Filtrat ein. Der Rückstand wird in Ether aufgenommen, die Lösung zweimal mit 0,5N Natronlauge gewaschen, getrocknet und eingedampft. Das zurückbleibende Öl wird mit Toluol an Kieselgel chromatographiert. Man erhält 5,3 g (86% d.Th.) 7-(4-Chlorphenyl)-2-(4-methylphenoxy)heptansäuremethylester, farbloses Öl.

e) Man erhitzt ein Gemisch von 3,25 g (9 mmol) 7-(4-Chlorphenyl)-2-(4-methylphenoxy)heptansäuremethylester, 15 ml 1 N Kalilauge und 45 ml Methanol unter Rühren 3 h auf 40° C. Dann dampft man im Vakuum das Methanol ab, wäscht die wässerige Lösung mit Ether und säuert sie mit verdünnter Salzsäure an. Das abgeschiedene Öl wird in Ether aufgenommen und die Etherlösung getrocknet und eingedampft. Den Rückstand reibt man mit Ligroin an. Man erhält 2,7 g (86% d.Th.) 7-(4-Chlorphenyl)-2-(4-methylphenoxy)-heptansäure, Fp. 74-76° C.

*Beispiel 10*

*7-(4-Chlorphenyl)-2-(4-methylphenylthio)heptansäuremethylester*

Nach der in Beispiel 9d) beschriebenen Arbeitsweise erhält man aus 6,0 g (18 mmol) 2-Brom-7-(4-chlorphenyl)heptansäuremethylester, 2,23 g (18 mmol) p-Thiokresol, 7,4 g (54 mmol) Kaliumcarbonat, 75 ml Butanon und 3,7 g (54% d.Th.) 7-(4-Chlorphenyl)-2-(4-methylphenylthio)heptansäuremethylester, farbloses Öl.

*Beispiel 11*

*7-(4-Chlorphenyl)-2-(4-methylphenylthio)heptansäure*

Man versetzt bei 0° C unter Stickstoff eine Lösung von 3,27 g (26,3 mmol) p-Thiokresol in 50 ml Ethanol mit einer Lösung von 2,12 g (53 mmol) Ätznatron in 5 ml Wasser und rührt 10 min nach. Dann fügt man eine Lösung von 8,4 g (26,3 mmol) 2-Brom-7-(4-chlorphenyl)heptansäure in 5 ml Ethanol zu und entfernt das Kühlbad. Anschliessend rührt man noch 6 h bei Zimmertemperatur, lässt über Nacht stehen und dampft ein. Den Rückstand nimmt man in Wasser auf, wäscht mit Ether und säuert mit Salzsäure an. Das abgeschiedene Öl wird mit Methylenchlorid extrahiert. Die Extrakte werden getrocknet und eingedampft. Den Rückstand chromatographiert man mit einem Gemisch von Toluol und Dioxan (5:1) an Kieselgel. Ausbeute: 6,6 g (69% d.Th) 7-(4-Chlorphenyl)-2-(4-methylphenylthio)heptansäure, Fp. 58-61° C.

*Beispiel 12*

*7-(4-Chlorphenyl)-2-[(4-methylphenyl)sulfinyl]heptansäureethylester*

Man versetzt eine Lösung von 8,5 g (37,6 mmol) 4-Methylphenylsulfinylessigsäureethylester in 75 ml Dimethylsulfoxid zunächst mit 1,64 g (37,6 mmol) Natriumhydrid in 55proz. Mineralölsuspension und dann mit 9,8 g (37,6 mmol) 5-(4-Chlorphenyl)pentylbromid. Anschliessend wird die Mischung noch 4 h bei Zimmertemperatur gerührt. Dann giesst man in Wasser, extrahiert mit Ether, wäscht die Extrakte mit Wasser, trocknet und dampft sie ein. Der Rückstand wird mit einem Gemisch von n-Heptan und Butanon (2:1) an Kieselgel chromatographiert. Ausbeute: 8,0 g (52% d.Th.) 7-(4-Chlorphenyl)-2-[(4-methylphenyl)-sulfinyl]heptansäureethylester, farbloses Öl.

*Beispiel 13*

*2-[(4-Methylphenyl)sulfonyl]octansäureethylester*

Nach der in Beispiel 1a) beschriebenen Arbeitsweise erhält man aus 40 mmol Natriumethylat

9,7 g (40 mmol) (4-Methylphenyl)sulfonylessigsäureethylester und 7,26 g (44 mmol) n-Hexylbromid in 100 ml Ethanol 7,3 g (56% d.Th.) 2-[(4-Methylphenyl)sulfonyl]octansäureethylester, farbloses Öl.

In analoger Weise erhält man aus (4-Methylphenyl)sulfonylessigsäureethylester und

a) n-Octylbromid:

2-[(4-Methylphenyl)sulfonyl]decansäureethylester, farbloses Öl, Ausb. 52% d.Th.

Der *Methylester* der zugrundeliegenden Säure ist bereits von Ono e.a., Bull. Chem. Soc. Jpn. *1979*, *52*, 1716, als farbloses Öl beschrieben worden.

b) n-Decylbromid:

2-[(4-Methylphenyl)sulfonyl]dodecansäureethylester, farbloses Öl, Ausbeute 57% d.Th.

c) n-Tetradecylbromid:

2-[(4-Methylphenyl)sulfonyl]hexadecansäureethylester, Fp. 38-40° C, Ausb. 68% d.Th.

Der *Methylester* der zugrundeliegenden Säure ist bereits von Engel u. Cowburn, J. Biomol. Struct. Dyn. *1983*, *1*, 319, beschrieben worden.

*Beispiel 14*

*2-[(4-Methylphenyl)sulfonyl]octansäure*

Nach der in Beispiel 1b) beschriebenen Arbeitsweise erhält man aus 4,75 g (14,5 mmol) 2-[(4-Methylphenyl)sulfonyl]octansäureethylester (Beispiel 13) und 32 ml 1N Kalilauge in 100 ml Methanol 2,6 g (60% d.Th.) 2-[8(4-Methylphenyl)sulfonyl]octansäure, Fp. 111-123° C (Essigsäureethylester).

In analoger Weise erhält man aus

a) 2-[(4-Methylphenyl)sulfonyl]decansäureethylester (Beispiel 13a):

2-[(4-Methylphenyl)sulfonyl]decansäure, Fp. 116-118° C, Ausb. 77% d.Th.

b) 2-[(4-Methylphenyl)sulfonyl]dodecansäureethylester (Beispiel 13b):

2-[(4-Methylphenyl)sulfonyl]dodecansäure, Fp. 71-73° C (Ligroin) Ausbeute 92% d.Th.

Diese Verbindung ist bereits von Takur und Nargund, Indian J. Chem., Sect. B *1977*, *15B*, 286 als potentielles Tuberkulostatikum beschrieben worden.

c) 2-[(4-Methylphenyl)sulfonyl]hexandecansäureethylester (Beispiel 13c):

2-[(4-Methylphenyl)sulfonyl]hexadecansäure, Fp. 87-90° C, Ausb. 93% d.Th.

*Beispiel 15*

*2-[(4-Methyphenyl)sulfonyl]-4-methyl-5-phenyl-4-pentensäure*

Nach der im Beispiel 1a) beschriebenen Arbeitsweise erhält man aus 9,69 g (40 mmol) (4-Methylphenyl)sulfonylessigsäureethylester, 40 mmol Natriumethylat und 6,66 g (40 mmol) 2-Methyl-3-phenyl-2-pentenylchlorid in 100 ml abs Ethanol:

8,2 g (55% d.Th.) 2-[(4-Methylphenyl)sulfonyl]-4-methyl-5-phenyl-4-pentensäureethylester, Fp. 123-124° C (Essigsäureethylester/Ligroin) BM 13.889 und daraus nach der in Beispiel 1b) beschriebenen Arbeitsweise 6,8 g (90% d.Th.) 2-[(4-Methylphenyl)sulfonyl]-4-methyl-5-phenyl-4-pentensäure, klebrige Kristalle. Das Natriumsalz schmilzt bei 140-142° C.

In analoger Weise erhält man aus

a) (4-Methylphenyl)sulfonylessigsäureethylester und 5-Phenyl-4-pentenylbromid:

2-[(4-Methylphenyl)sulfonyl]-7-phenyl-6-heptensäureethylester, Fp. 46-49° C, Ausb. 56%, und daraus

2-[(4-Methylphenyl)sulfonyl]-7-phenyl-6-heptensäure, farbloses Öl, Ausb. 75%, Natriumsalz: Fp. 144° C (Z)

b) (4-Methylphenyl)sulfonylessigsäureethylester und 5-(4-Methylphenyl)pentylbromid:

2-[(4-Methylphenyl)sulfonyl]-7-(4-methylphenyl)heptansäureethylester, farbloses Öl, Ausb. 67%,

und daraus

2-[(4-Methylphenyl)sulfonyl]-7-(4-methylphenyl)heptansäure, farbloses Öl, Ausb. 83%

c) (4-Methylphenyl)sulfonylessigsäureethylester und 4-(4-Chlorphenyl)butylbromid:

6-(4-Chlorphenyl)-2-[(4-methylphenyl)sulfonyl]hexansäureethylester, farbloses Öl, Ausb. 74%,

und daraus

6-(4-Chlorphenyl)-2-[(4-methylphenyl)sulfonyl]hexansäure, farbloses Öl, Ausb. 99%, Natriumsalz: Fp. 143-145° C

d) (3-Chlorphenyl)sulfonylessigsäureethylester und 4-(4-Chlorphenyl)pentylbromid:

7-(4-Chlorphenyl)-2-[(4-chlorphenyl)sulfonyl]-heptansäureethylester, farbloses Öl, Ausb. 69%,

und daraus

7-(4-Chlorphenyl)-2-[(3-chlorphenyl)sulfonyl]heptansäure, Fp. 104-106° C, Ausb. 73%

e) (4-Methoxyphenyl)sulfonylessigsäureethylester und 5-(4-Chlorphenyl)pentylbromid:

7-(4-Chlorphenyl)-2-[(4-methoxyphenyl)sulfonyl]heptansäureethylester, farbloses Öl, Ausb. 79%,

und daraus

7-(4-Chlorphenyl)-2-[(4-methoxyphenyl)sulfonyl]heptansäure, Fp. 58-65° C, Ausb. 64%

f) (3-Trifluormethylphenyl)sulfonylessigsäureethylester und 5-(4-Chlorphenyl)pentylbromid:

7-(4-Chlorphenyl)-2-[(3-trifluormethylphenyl)sulfonyl]heptansäureethylester, farbloses Öl, Ausb. 52%,

und daraus

7-(4-Chlorphenyl)-2-[(3-trifluormethylphenyl)sulfonyl]heptansäure, Fp. 107-108° C, Ausb. 73%, Natriumsalz: Fp. 147-150° C

g) (4-Cyanophenyl)sulfonylessigsäureethylester und 5-(4-Chlorphenyl)pentylbromid:

7-(4-Chlorphenyl)-2-[(4-cyanophenyl)sulfonyl]heptansäureethylester, farbloses Öl, Ausb. 65%

h) (4-Acetylphenyl)sulfonylessigsäureethylester und 5-(4-Chlorphenyl)pentylbromid:

2-[(4-Acetylphenyl)sulfonyl]-7-(4-chlorphenyl)heptansäureethylester, farbloses Öl, Ausb. 54%

i) (1-Naphthyl)sulfonylessigsäureethylester und 5-(4-Chlorphenyl)pentylbromid:

7-(4-Chlorphenyl)-2-[(1-naphthyl)sulfonyl]-heptansäureethylester, farbloses Öl, Ausb. 71%,
und daraus

7-(4-Chlorphenyl)-2-[(1-naphthyl)sulfonyl]-heptansäure, farbloses Öl, Ausb. 63%, Natriumsalz: Fp. 168-170° C

j) (4-Methylphenyl)sulfonylessigsäureethylester und 6-(4-Chlorphenyl)hexylbromid:

8-(4-Chlorphenyl)-2-[(4-methylphenyl)sulfonyl]octansäureethylester, farbloses Öl, Ausb. 67%,
und daraus

8-(4-Chlorphenyl)-2-[(4-methylphenyl)sulfonyl]octansäure, farbloses Öl, Ausb. 71%

k) (4-Methylphenyl)sulfonylessigsäureethylester und 4-(4-Chlorphenoxy)butylbromid:

6-(4-Chlorphenoxy)-2-[(4-methylphenyl)sulfonyl]hexansäureethylester, farbloses Öl, Ausb. 67%,
und daraus

6-(4-Chlorphenoxy)-2-[(4-methylphenyl)sulfonyl]hexansäure, farbloses Öl, Ausb. 82%, Natriumsalz: Fp. 168-171° C

l) (4-Methylphenyl)sulfonylessigsäureethylester und 5-(4-Chlorphenoxy)pentylbromid:

7-(4-Chlorphenoxy)-2-[(4-methylphenyl)sulfonyl]heptansäureethylester, Fp. 86-89° C, Ausb. 63%,
und daraus

7-(4-Chlorphenoxy)-2-[(4-methylphenyl)sulfonyl]heptansäure, Fp. 88-90° C, Ausb. 87%

m) (4-Methylphenyl)sulfonylessigsäureethylester und 5-(4-Methoxyphenyl)pentylbromid:

7-(4-Methoxyphenyl)-2-[(4-methylphenyl)sulfonyl]heptansäureethylester, Fp. 64-68° C, Ausb. 56%,
und daraus

7-(4-Methoxyphenyl)-2-[(4-methylphenyl)sulfonyl]heptansäure, farbloses Öl, Ausb. 70%, Natriumsalz: Fp. 72-80° C (Zers.)

n) (4-Methylphenyl)sulfonylessigsäureethylester und 5-(3-Trifluormethylphenyl)pentylbromid:

2-[(4-Methylphenyl)sulfonyl]-7-(3-trifluormethylphenyl)heptansäureethylester, Fp. 56-58° C, Ausb. 53%,
und daraus

2-[(4-Methylphenyl)sulfonyl]-7-(3-trifluormethylphenyl)heptansäure, Fp. 62° C, Ausb. 87%

o) (4-Methylphenyl)sulfonylessigsäureethylester und 5-(2-Methoxyphenyl)pentylbromid:

7-(2-Methoxyphenyl)-2-[(4-methylphenyl)sulfonyl]heptansäureethylester, farbloses Öl, Ausb. 64%,
und daraus

7-(2-Methoxyphenyl)-2-[(4-methylphenyl)sulfonyl]heptansäure, farbloses Öl, Ausb. 70%, Natriumsalz: Fp. 75-76° C

p) 4-(Methylphenyl)sulfonylessigsäureethylester und 5-(3-Methoxyphenyl)pentylbromid:

7-(3-Methoxyphenyl)-2-[(4-methylphenyl)sulfonyl]heptansäureethylester, farbloses Öl, Ausb. 52%,
und daraus

7-(3-Methoxyphenyl)-2-[(4-methoxyphenyl)-sulfonyl]heptansäure, farbloses Öl, Ausb. 60%, Natriumsalz: Fp. 84-85° C

q) (4-Methylphenyl)sulfonylessigsäureethylester und 3-(4-Chlorphenyl)-2-propinbromid:

5-(4-Chlorphenyl)-2-[(4methylphenyl)sulfonyl]-4-pentinsäureethylester, Fp. 78-79° C, Ausb. 57%,
und daraus

5-(4-Chlorphenyl)-2-[(4-methylphenyl)sulfonyl]-4-pentinsäure, Fp. 154-157° C, Ausb. 76%

*Beispiel 16*

*2-(Aminocarbonyl)-7-(4-chlorphenyl)heptansäure*

a) Man versetzt ein Gemisch von 9,0 g (34,4 mmol) 5-(4-Chlorphenyl)pentylbromid, 5,2 g (34,4 mmol) 1,8-Diazabicyclo-(5,4,0)-undec-7-en und 18 ml wasserfreies Benzol unter Rühren mit einer Lösung von 3,9 g (34,4 mmol) Cyanessigsäureethylester in 36 ml wasserfreiem Benzol und rührt das Gemisch noch 16 h bei Zimmertemperatur nach. Dann wäscht man die organische Phase mit Wasser, trocknet und engt ein. Der Rückstand wird mit einem Gemisch von n-Heptan und Butanon (2:1) an Kieselgel chromatographiert. Man erhält 8,8 g (87% d.Th.) 7-(4-Chlorphenyl)-2-cyanoheptansäureethylester, farbloses Öl.

b) 12 g (41 mmol) 7-(4-Chlorphenyl)-2-cyanoheptansäureethylester und 96 g Polyphosphorsäure werden unter Rühren 3 Stunden auf 100° C erhitzt. Dann wird mit 200 ml Wasser verdünnt und das Gemisch dreimal mit Methylenchlorid extrahiert. Die vereinigten Extrakte werden mit Wasser gewaschen, getrocknet und eingeengt. Man erhält 10 g (78% d.Th) 2-(Aminocarbonyl)-7-(4-chlorphenyl)heptansäureethylester, Fp. 110-111° C.

c) Ein Gemisch von 7,8 g (25 mmol) 2-(Aminocarbonyl)-7-(4-chlorphenyl)heptansäure-ethylester, 25 ml 1 N Kalilauge und 100 ml Methanol wird 4 h bei 40° C gerührt. Dann dampft man das Methanol ab, kohlt den wässerigen Rückstand und säuert ihn mit 2N Salzsäure an. Man extrahiert die abgeschiedene Rohsäure mit Methylenchlorid, trocknet die Extrakte und dampft sie ein. Den Rückstand kristallisiert man aus einem Gemisch von Essigester und Ligroin um. Ausbeute: 4,8 g (68% d.Th.) 2-(Aminocarbonyl)-7-(4-chlorphenyl)heptansäure, Fp. 125-126° C

In analoger Weise erhält man
aus 3-(4-Chlorphenyl)propylbromid und Cyanessigsäureethylester:

a-1) 5-(4-Chlorphenyl)-2-cyanopentansäureethylester, farbloses Öl, Ausb. 74% d.Th.

b-1) 2-(Aminocarbonyl)-5-(4-chlorphenyl)-heptansäureethylester, Fp. 99-101° C (Essigester/Ligroin)
aus 6-(4-Chlorphenyl)hexylbromid und Cyanessigsäureethylester

a-2) 8-(4-Chlorphenyl)-2-cyanooctansäureethylester, farbloses Öl, Ausb. 87% d.Th.

b-2) 2-(Aminocarbonyl)-8-(4-chlorphenyl)-octansäureethylester, Fp. 121-122° C (Essigester/Ligroin)

*Beispiel 17*

*2-Acetyl-7-(4-chlorphenyl)heptansäureethylester*

Zu einer Lösung von 23 mmol Natriumethylat in 18 ml Ethanol tropft man unter Rühren eine Lösung von 6,0 g (46 mmol) Acetessigsäureethylester in 6 ml abs. Ethanol. Man lässt noch 15 min zur Vervollständigung der Salzbildung nachrühren und fügt dann eine Lösung von 6,0 g (23 mmol) 5-(4-Chlorphenyl)pentylbromid in 6 ml abs. Ethanol zu. Das Gemisch wird nun 3 Stunden unter Rückfluss erhitzt und anschliessend vollständig eingedampft. Den Rückstand versetzt man mit Wasser und neutralisiert das Gemisch mit 2N Salzsäure. Dann extrahiert man die organischen Bestandteile mit Ether, trocknet die vereinigten Extrakte und dampft sie ein. Der Rückstand wird mit einem Gemisch von n-Heptan und Butanon an Kieselgel chromatographiert. Ausbeute: 5,8 g (81% d.Th.) 2-Acetyl-7-(4-chlorphenyl)heptansäureethylester, farbloses Öl.

*Beispiel 18*

*5-(4-Chlorphenyl)-2-[(3-(4-chlorphenyl)-2-propin-1-yl]-2-[(4-methylphenyl)sulfonyl]-4-pentinsäureethylester*

Nach der in Beispiel 1 a) beschriebenen Arbeitsweise erhält man aus 7,8 g (34 mmol) (4-Methylphenyl)sulfonylessigsäureethylester, 68 mmol Natriumethylat und 16,5 g (68 mmol) 3-(4-Chlorphenyl)-2-propin-1-ylbromid 8,1 g (44% d.Th.) der Titelverbindung, Fp. 90-93° C.

*Beispiel 19*

*2-(Aminocarbonyl)-7-(4-chlorphenyl)heptansäureethylester*

Zu einer Lösung von 5,6 g (17 mmol) 2-(Chlorcarbonyl)-7-(4-chlorphenyl)heptansäureethylester in 50 ml Methylenchlorid tropft man bei −10° C eine gesättigte Lösung von gasförmigem Ammoniak in 25 ml Methylenchlorid. Man rührt 30 min bei Zimmertemperatur, saugt vom Ammoniumchlorid ab, wäscht das Filtrat mit Wasser, trocknet und dampft ein. Der Rückstand wird mit einem Gemisch von Toluol und Dioxan (5:1) an Kieselgel chromatographiert. Man erhält 3,6 g (68% d.Th.) 2-(Aminocarbonyl)-7-(4-chlorphenyl)heptansäureethylester, Fp. 110-111° C.

*Beispiel 20*

Durch geeignete Wahl von Ausgangskomponenten und Reaktionsbedingungen lassen sich in Anlage zu den vorangegangenen Beispielen herstellen:

7-(4-Chlorphenyl)-2-(methoxy)heptansäure
7-(4-Chlorphenyl)-2-(methylthio)heptansäure
7-(4-Chlorphenyl)-2-(methylsulfoxyl)heptansäure
7-(4-Chlorphenyl)-2-(phenoxy)heptansäure
7-(4-Chlorphenyl)-2-(4-chlorphenyl)heptansäure
7-(4-Chlorphenyl)-2-(4-methoxyphenoxy)-heptansäure
7-(4-Chlorphenyl)-2-(3-trifluormethylphenoxy)heptansäure
7-(4-Chlorphenyl)-2-(4-cyanophenoxy)heptansäure
2-(4-Methoxyphenoxy)-7-phenylheptansäure
7-(4-Methoxyphenyl)-2-(4-methylphenoxy)-heptansäure
2-(4-Methylphenoxy)-7-(3-trifluormethylphenyl)heptansäure
7-(4-Chlorphenyl)-2-(phenylthio)heptansäure
7-(4-Chlorphenyl)-2-[(4-chlorphenyl)thio]-heptansäure
7-(4-Chlorphenyl)-2-[(4-methoxyphenyl)thio]-heptansäure
7-(4-Chlorphenyl)-2-[(3-trifluormethylphenyl)-thio]heptansäure
7-(4-Chlorphenyl)-2-[(4-cyanophenyl)thio]-heptansäure
2-[(4-Methylphenyl)thio]-7-phenylheptansäure
7-(4-Methoxyphenyl)-2-[(4-methylphenyl)-thio]heptansäure
2-[(4-Methylphenyl)thio]-7-(3-trifluormethylphenyl)heptansäure
7-(4-Chlorphenyl)-2-[(phenyl)sulfinyl]heptansäure
7-(4-Chlorphenyl)-2-[(4-chlorphenyl)sulfinyl]-heptansäure
7-(4-Chlorphenyl)-2-[(4-methoxyphenyl)-sulfinyl]heptansäure
7-(4-Chlorphenyl)-2-[(3-trifluormethylphenyl)-sulfinyl]heptansäure
7-(4-Chlorphenyl)-2-[(4-cyanophenyl)-sulfinyl]heptansäure
2-[(4-Methylphenyl)sulfinyl]-7-phenylheptansäure
7-(4-Methoxyphenyl)-2-[(4-methylphenyl)-sulfinyl]heptansäure
2-[(4-Methylphenyl)sulfinyl]-7-(3-trifluormethylphenyl)heptansäure
6-Cyclohexyl-2-[(4-methylphenyl)sulfonyl]-heptansäure
8-Cyclohexyl-2-[(4-methylphenyl)sulfonyl]-octansäure
8-Methoxy-2-[(4-methylphenyl)sulfonyl]-octansäure
8-Methylthio-2-[(4-methylphenyl)sulfonyl]-octansäure
8-(Methylsulfinyl)-2-[(4-methylphenyl)sulfonyl]octansäure
4-Methyl-2-[(4-methylphenyl)sulfonyl]-5-phenylpentansäure
2-[(4-Methylphenyl)sulfonyl]-5-phenyl-4-pentinsäure
2-[(4-Methylphenyl)sulfonyl]-6-phenylhexansäure

7-Phenyl-2-(phenylsulfonyl)heptansäure
2-[(4-Chlorphenyl)sulfonyl]-7-phenylheptansäure
2-[(4-Methoxyphenyl)sulfonyl]-7-phenylheptansäure
2-[(4-Cyanophenyl)sulfonyl]-7-phenylheptansäure
7-Phenyl-2-[(3-trifluormethylphenyl)sulfonyl]-heptansäure
7,7-Dimethyl-2-[(4-methylphenyl)sulfonyl]-7-phenylheptansäure
2-[(4-Methylphenyl)sulfonyl]-8-phenyloctansäure
6-(4-Methylphenyl)-2-[(4-methylphenyl)-sulfonyl]hexansäure
8-(4-Methylphenyl)-2-[(4-methylphenyl)-sulfonyl]octansäure
10-(4-Methylphenyl)-2-[(4-methylphenyl)-sulfonyl]decansäure
7-(4-Chlorphenyl)-2-(octylsulfonyl)heptansäure
7-(4-Chlorphenyl)-2-[(4-nitrophenyl)sulfonyl]-heptansäure
7-(4-Chlorphenyl)-2-[(4-dimethylaminophenyl)sulfonyl]heptansäure
7-(4-Chlorphenyl)-2-[(4-hydroxyphenyl)-sulfonyl]heptansäure
7-(4-Chlorphenyl)-2-[(2-naphthyl)sulfonyl]-heptansäure
9-(4-Chlorphenyl)-2-[(4-methylphenyl)-sulfonyl]nonansäure
10-(4-Chlorphenyl)-2-[(4-methylphenyl)-sulfonyl]decansäure
7-(4-Chlorphenyl)-2-[(2-phenylethyl)sulfonyl]heptansäure
7-(4-Chlorphenyl)-2-[(2-phenylethenyl)sulfonyl]heptansäure
7-(4-Chlorphenyl)-2-[(5-(4-chlorphenyl)-pentyl)sulfonyl]heptansäure
7-(4-Chlorphenyl)-2-[(4-methylphenyl)sulfonyl]-6-heptensäure
5-(4-Methoxyphenyl)-2-[(4-methylphenyl)-sulfonyl]-4-pentinsäure
2-[(4-Methylphenyl)sulfonyl]-5-(3-trifluormethylphenyl)-4-pentinsäure
5-(4-Chlorphenyl)-2-(phenylsulfonyl)-4-pentinsäure
5-(4-Chlorphenyl)-2-[(4-chlorphenyl)sulfonyl]-4-pentinsäure
5-(4-Chlorphenyl)-2-[(3-trifluormethylphenyl)sulfonyl]-4-pentinsäure
5-(4-Chlorphenyl)-2-[(4-methoxyphenyl)-sulfonyl]-4-pentinsäure
5-(4-Chlorphenyl)-2-[(4-cyanophenyl)sulfonyl]-4-pentinsäure
7-(4-Methoxyphenyl)-2-(phenylsulfonyl)-heptansäure
2-[(4-Chlorphenylsulfonyl]-7-(4-methoxyphenyl)heptansäure
7-(4-Methoxyphenyl)-2-[(4-methoxyphenyl)-sulfonyl]heptansäure
2-(Phenylsulfonyl)-7-(3-trifluormethylphenyl)-heptansäure
2-[(4-Chlorphenyl)sulfonyl]-7-(3-trifluormethylphenyl)heptansäure
2-[(4-Methoxyphenyl)sulfonyl]-7-(3-trifluormethylphenyl)heptansäure
7-(3,4-Dichlorphenyl)-2-(phenylsulfonyl)-heptansäure
7-(3,4-Dichlorphenyl)-2-[(4-methylphenyl)-sulfonyl]heptansäure
2-[(4-Chlorphenyl)sulfonyl]-7-(3,4-dichlorphenyl)heptansäure
7-(3,4-Dichlorphenyl)-2-[(4-methoxyphenyl)-sulfonyl]heptansäure
7-(4-Fluorphenyl)-2-[(4-methylphenyl)sulfonyl]heptansäure
7-(3-Chlorphenyl)-2-[(4-methylphenyl)sulfonyl]heptansäure
7-(4-Aminophenyl)-2-[(4-methylphenyl)sulfonyl]heptansäure
2-[(4-Methylphenyl)sulfonyl]-7-[4-nitrophenyl)heptansäure
2-[(4-Methylphenyl)sulfonyl]-7-(1-naphthyl)-heptansäure
2-[(4-Methylphenyl)sulfonyl]-7-(2-naphthyl)-heptansäure
2-(Acetoxy)-7-(4-chlorphenyl)heptansäure
2-(Benzoyloxy)-7-(4-chlorphenyl)heptansäure
2-Amino-7-(4-chlorphenyl)heptansäure
2-Amino-8-(4-chlorphenyl)octansäure
2-Amino-7-(4-methoxyphenyl)heptansäure
2-Amino-7-(3-trifluormethylphenyl)heptansäure
2-Acetylamino-7-(4-chlorphenyl)heptansäure
2-(Benzoylamino)-7-(4-chlorphenyl)heptansäure
7-(4-Chlorphenyl)-2-(diethylamino)heptansäure
7-(4-Chlorphenyl)-2-(phenylamino)heptansäure
2-(Benzylamino)-7-(4-chlorphenyl)heptansäure
7-(4-Chlorphenyl)-2-piperidinoheptansäure
7-(4-Chlorphenyl)-2-morpholinoheptansäure
7-(4-Chlorphenyl)-2-(4-Phenylpiperazino)-heptansäure
2-(Aminosulfonyl)-7-(4-chlorphenyl)heptansäure
7-(4-Chlorphenyl)-2-[(diethylamino)sulfonyl]-heptansäure
7-(4-Chlorphenyl)-2-[(Piperidino)sulfonyl]-heptansäure
2-(Aminocarbonyl)-5-(4-chlorphenyl)-4-pentensäure
2-(Aminocarbonyl)-7-(4-chlorphenyl)-2-methylheptansäure
2-(Aminocarbonyl)-7-phenylheptansäure
2-(Aminocarbonyl)-7-(4-methoxyphenyl)-heptansäure
2-(Aminocarbonyl)-7-(4-methylphenyl)heptansäure
2-(Aminocarbonyl)-7-(3-trifluormethylphenyl)-heptansäure
7-Phenyl-2-(phenoxy)heptansäure
2-(4-Methoxyphenoxy)-7-phenylheptansäure
2-(3-Trifluormethylphenoxy)-7-phenylheptansäure
2-(4-Cyanophenoxy)-7-phenylheptansäure
2-(4-Methylphenoxy)-8-phenyloctansäure

2-(4-Methylphenoxy)-10-phenyloctansäure
8-(4-Chlorphenyl)-2-(4-methylphenoxy)octansäure
10-(4-Chlorphenyl)-2-(4-methylphenoxy)-decansäure
7-(2-Methoxyphenyl)-2-(4-methylphenoxy)-heptansäure
8-(2-Methoxyphenyl)-2-(4-methylphenoxy)-octansäure
7-(5-Chlor-2-methoxyphenyl)-2-(4-methylphenoxy)heptansäure
7-Phenyl-2-(phenylthio)heptansäure
2-[(4-Chlorphenyl)thio]-7-phenylheptansäure
2-[(4-Methoxyphenyl)thio]-7-phenylheptansäure
7-Phenyl-2-[(3-trifluormethylphenyl)thio]-heptansäure
2-[(4-Cyanophenyl)thio]-7-phenylheptansäure
2-[(4-Methylphenyl)thio]-8-phenyloctansäure
2-[(4-Methylphenyl)thio]-10-phenyldecansäure
8-(4-Chlorphenyl)-2-[(4-methylphenyl)thio]-octansäure
10-(4-Chlorphenyl)-2-[(4-methylphenyl)-thio]decansäure
7-(2-Methoxyphenyl)-2-[(4-methylphenyl)-thio]heptansäure
8-(2-Methoxyphenyl)-2-[(4-methylphenyl)-thio]octansäure
7-(5-Chlor-2-methoxyphenyl)-2-[(4-methylphenyl)thio]heptansäure
5-phenyl-2-(phenylsulfonyl)-4-pentinsäure
2-[(4-Chlorphenyl)sulfonyl]-5-phenyl-4-pentinsäure
5-Phenyl-2-[(3-trifluormethylphenyl)sulfonyl]-4-pentinsäure
2-[(4-Methoxyl)sulfonyl]-5-phenyl-4-pentinsäure
2-[(4-Cyanophenyl)sulfonyl]-5-phenyl-4-pentinsäure
2-[(4-Methylphenyl)sulfonyl]-7-phenyl-6-heptinsäure
5-(2-Methoxyphenyl)-2-[(4-methylphenyl)-sulfonyl]-4-pentinsäure
5-(5-Chlor-2-methoxyphenyl)-2-[(4-methylphenyl)sulfonyl]-4-pentinsäure
7-(4-Chlorphenyl)-3,3-dimethyl-2-[(4-methylphenyl)sulfonyl]heptansäure
8-(4-Chlorphenyl)-3,3-dimethyl-2-[(4-methylphenyl)sulfonyl]octansäure
10-(4-Chlorphenyl)-3,3-dimethyl-2-[(4-methylphenyl)sulfonyl]decansäure
7-(4-Chlorphenyl)-3,3-dimethyl-2-[(4-methylphenyl)thio]heptansäure
8-(4-Chlorphenyl)-3,3-dimethyl-2-[(4-methylphenyl)thio]octansäure
10-(4-Chlorphenyl)-3,3-dimethyl-2-[(4-methylphenyl)thio]decansäure
7-(4-Chlorphenyl)-3,3-dimethyl-2-(4-methylphenoxy)heptansäure
8-(4-Chlorphenyl)-3,3-dimethyl-2-(4-methylphenoxy)octansäure
10-(4-Chlorphenyl)-3,3-dimethyl-2-(4-methylphenoxy)decansäure
7-Phenyl-2-[(2-phenylethyl)sulfonyl]heptansäure

## Patentansprüche

1. Carbonsäurederivate der allgemeinen Formel I

$$R_1-A-\underset{X}{\overset{R_2}{C}}-COOH \qquad (I)$$

in welcher

$R_1$ einen gegebenenfalls substituierten Aryl- oder Aryloxyrest,

$R_2$ Wasserstoff, einen niederen Alkylrest oder die Gruppe $R_1-A-$, sowie für den Fall, dass X die Cyanogruppe bedeutet, eine Acylamino- oder Aminogruppe,

A eine geradkettige oder verzweigte, gesättigte oder ungesättigte Alkylengruppe mit 3-8 Kohlenstoffatomen, die eine Kettenlänge von mindestens 3 Kohlenstoffatomen besitzt,

X die Cyanogruppe oder eine Gruppe der Formel $-B-R_3$ oder $-D-NR_4R_5$,

in welcher

B O, S, SO, $SO_2$, O(CO), $OSO_2$, NHCO, $NHSO_2$ oder CO,

D einen Valenzstrich, $SO_2$ oder CO,

$R_3$ eine Alkyl, Trifluormethyl-, Cycloalkyl-, Aralkyl-, Aralkenyl- oder Arylgruppe, wobei der jeweilige Arylrest gegebenenfalls asubstituiert sein kann,

$R_4$ Wasserstoff, einen niederen Alkylrest, einen gegebenenfalls substituierten Aryl- oder Aralkylrest,

$R_5$ Wasserstoff, einen niederen Alkylrest oder

$R_4$ u. $R_5$ gemeinsam eine Alkylenkette mit 4-6 Kohlenstoffatomen, die durch O, S oder $NR_6$ unterbrochen sein kann, und

$R_6$ Wasserstoff, einen niederen Alkylrest oder einen gegebenenfalls substituierten Phenyl- oder Benzylrest bedeutet,

sowie deren physiologisch unbedenklichen Salze, Ester, Amide und Nitrile,

mit der Massgabe, dass für den Fall,

a) dass A eine Alkylengruppe mit 3 Kohlenstoffatomen ist,

$a_1$) in allen Fällen der Arylrest der Gruppe $R_1$ nicht den unsubstituierten Phenylrest und

$a_2$) X nicht die Gruppen $-CN$, $-NHCOR_3$ und $NR_4R_5$,

b) dass X ein Rest der Formel $-SCH_3$ ist, $R_2$ nicht Methyl,

c) dass X die Gruppe $-NH_2$ oder $-NHCOCH_3$ ist,

$R_1A-$ nicht 4-Phenylbutyl oder 4-(4-Methoxyphenyl)butyl,

d) dass X die Gruppe 2,4-Dinitrophenyl ist, $R_1A-$ nicht 4-Phenylbutyl oder 5-Phenylpentyl,

e) dass X die Gruppe $-COCH_3$ ist,

$R_1A-$ nicht 3-(2-Chlorphenyl)propyl oder 5-(4-Methoxyphenyl)pentyl

bedeuten darf.

2. Carbonsäurederivate der allgemeinen Formel I gemäss Anspruch 1, in welcher

$R_1$ einen Phenyl-, Naphthyl-, Phenyloxy- oder Naphthyloxyrest bedeutet, der ein oder mehrfach durch Hydroxyl-, Halogen-, Alkyl-, Alkoxy-, Trifluormethyl-, Cyano-, Nitro-, Amino, Alkylamino und Dialkylaminogruppen substituiert sein kann,

$R_2$ Wasserstoff, eine Methyl- oder Ethylgruppe oder die Gruppe $R_1-A$ bedeutet,

A eine geradkettige, gesättigte oder ungesättigte Alkylengruppe mit 3-8 Kohlenstoffatomen bedeutet,

X eine Cyano-, Alkoxycarbonyl-, Alkoxy-, Alkylsulfenyl-, Alkylsulfinyl-, Alkylsulfonyl-, Trifluormethylsulfonyl, Phenoxy-, Phenylsulfenyl-, Phenylsulfinyl-, Phenylsulfonyl-, Naphthylsulfonyl-, Phenylethylensulfonyl-, Phenylethenylensulfonyl-, Alkylcarbonyloxy-, Alkylsulfonyloxy-, Alkylcarbonylamino, Alkylsulfonylamino, Alkylcarbonyl, Phenylcarbonyloxy, Phenylsulfonyloxy, Phenylcarbonylamino, Phenylsulfonylamino, Phenylcarbonyl, Amino, Dialkylamino, Phenylamino, Benzylamino, Morpholino, Piperidino, 4-Benzylpiperazino, Aminosulfonyl, Dialkylaminosulfonyl, Piperidinosulfonyl, Carbamoyl, Dialkylaminocarbonyl, Phenylaminocarbonyl, Benzylaminocarbonyl, Piperidinocarbonyl-, Morpholinocarbonyl, 4-Phenylpiperazinocarbonyl und 4-Benzylpiperazinocarbonyl bedeutet, wobei der Phenylring in allen Fällen durch die oben genannten Substituenten substituiert sein kann.

3. Verfahren zur Herstellung von Carbonsäurederivaten der allgemeinen Formel I

$$\begin{array}{c} R_2 \\ | \\ R_1-A-C-COOH \\ | \\ X \end{array} \qquad (I)$$

in welcher

$R_1$ einen gegebenenfalls substituierten Aryl- oder Aryloxyrest,

$R_2$ Wasserstoff, einen niederen Alkylrest oder die Gruppe $R_1-A-$, sowie für den Fall, dass X die Cyanogruppe bedeutet, eine Acylamino- oder Aminogruppe,

A eine geradkettige oder verzweigte, gesättigte oder ungesättigte Alkylengruppe mit 3-8 Kohlenstoffatomen, die eine Kettenlänge von mindestens 3 Kohlenstoffatomen besitzt,

X die Cyanogruppe oder eine Gruppe der Formel $-B-R_3$ oder $-D-NR_4R_5$,

in welcher

B O, S, SO, $SO_2$, O(CO), $OSO_2$, NHCO, $NHSO_2$ oder CO,

D einen Valenzstrich, $SO_2$ oder CO,

$R_3$ eine Alkyl-, Trifluormethyl-, Cycloalkyl-, Aralkyl-, Aralkenyl- oder Arylgruppe, wobei der jeweilige Arylrest gegebenenfalls substituiert sein kann,

$R_4$ Wasserstoff, einen niederen Alkylrest, einen gegebenenfalls substituierten Aryl- oder Aralkylrest,

$R_5$ Wasserstoff, einen niederen Alkylrest oder

$R_4$ u. $R_5$ gemeinsam eine Alkylenkette mit 4-6 Kohlenstoffatomen, die durch O, S oder $NR_6$ unterbrochen sein kann, und

$R_6$ Wasserstoff, einen niederen Alkylrest oder einen gegebenenfalls substituierten Phenyl- oder Benzylrest bedeutet,

sowie deren physiologisch unbedenklichen Salze, Ester, Amide und Nitrile,

mit der Massgabe, dass für den Fall,

a) dass A eine Alkylengruppe mit 3 Kohlenstoffatomen ist,

$a_1$) in allen Fällen der Arylrest der Gruppe $R_1$ nicht den unsubstituierten Phenylrest und

$a_2$) X nicht die Gruppen $-CN$, $-NHCOR_3$ und $NR_4R_5$,

b) dass X ein Rest der Formel $-SCH_3$ ist, $R_2$ nicht Methyl,

c) dass X die Gruppe $-NH_2$ oder $-NHCOCH_3$ ist,

$R_1A-$ nicht 4-Phenylbutyl oder 4-(4-Methoxyphenyl)butyl,

d) dass X die Gruppe 2,4-Dinitrophenyl ist, $R_1A-$ nicht 4-Phenylbutyl oder 5-Phenylpentyl,

e) dass X die Gruppe $-COCH_3$ ist, $R_1A-$ nicht 3-(2-Chlorphenyl)propyl oder 5-(4-Methoxyphenyl)pentyl

bedeuten darf, dadurch gekennzeichnet, dass man

A) in an sich bekannter Weise eine Verbindung der allgemeinen Formel II,

$$\begin{array}{c} Z_1 \\ | \\ H-C-W \\ | \\ Z_2 \end{array} \qquad (II)$$

in welcher

$Z_1$ $-COOR_7$, $-CN$, $-CO-R_3$, $-SO-R_3$, $-SO_2-R_3$, $-CONH_2$ oder $-SO_2NH_2$,

$Z_2$ Wasserstoff, niederes Alkyl oder $-NH-R_8$

W $-COOR_7$ oder eine andere in die Carboxylfunktion überführbare Gruppe,

$R_7$ niederes Alkyl und

$R_8$ eine Aminoschutzgruppe

bedeuten und $R_2$ die oben angegebene Bedeutung hat, entweder

a1) mit einer Verbindung der allgemeinen Formel III,

$$R_1-A-Y \qquad (III)$$

in welcher $R_1$ und A die oben angegebenen Bedeutungen haben und Y einen reaktiven Rest darstellt, alkyliert oder

a2) für den Fall, dass $Z_2$ Wasserstoff bedeutet, mit einer Verbindung der allgemeinen Formel III'

$$R_1-A'-CHO \qquad (III')$$

in welcher A' einen um die $CH_2$-Gruppe verkürzten Alkylenrest A bedeutet und $R_1$ die oben angegebene Bedeutung besitzt, kondensiert und im Anschluss an die Kondensation die entstandene Doppelbindung hydriert und

b) die aus den vorgenannten Verfahrensschritten erhaltenen Verbindungen der allgemeinen Formel IV

$$R_1-A-\overset{\displaystyle Z_1 \atop |}{\underset{| \atop \displaystyle Z_2}{C}}-W \qquad (IV)$$

gegebenenfalls umwandelt, indem man z.B.

b1) für den Fall, dass $Z_2$ Wasserstoff bedeutet, mit einer Verbindung der allgemeinen Formel V,

$$R_2-Y \qquad (V)$$

in welcher $R_2$ die oben genannte Bedeutung besitzt und Y eine reaktive Gruppe darstellt, erneut alkyliert oder

b2) für den Fall, dass $Z_1$ $-COOR_7$ und $Z_2$ die Gruppe $-NH-R_8$ bedeuten,
den Rest $R_8$ nach an sich bekannten Methoden in die Reste $-CO-R_3$ oder $-SO_2-R_3$ überführt oder

b3) für den Fall, dass $Z_1$ $COOR_7$ und $Z_2$ Wasserstoff bedeuten,
das Wasserstoffatom in an sich bekannter Weise mit einem Halogenierungsmittel durch ein Halogenatom austauscht, im Anschluss daran den Rest $Z_1$ durch Decarboxylierung in ein Wasserstoffatom überführt und das reaktive Derivat IV,
in welchem nun für $Z_2$ Halogen und für $Z_1$ Wasserstoff steht,
entweder mit einer Gruppe der Formel VI,

$$H-B-R_3 \qquad (VI)$$

in welcher B für O, S oder $NHSO_2$ steht und $R_3$ die oben angegebene Bedeutung hat,
zu der Verbindung

$$R_1-A-\overset{\displaystyle H \atop |}{\underset{| \atop \displaystyle B-R_3}{C}}-W \qquad (IV')$$

oder mit einer Gruppe der Formel VI',

$$HNR_4R_5 \qquad (VI')$$

in welcher $R_4$ und $R_5$ die oben angegebene Bedeutung hat, zu der Verbindung

$$R_1-A-\overset{\displaystyle H \atop |}{\underset{| \atop \displaystyle NR_4R_5}{C}}-W \qquad (IV'')$$

c) im Anschluss an die Kondensation für den Fall, dass

c1) B ein Schwefelatom bedeutet, nach an sich bekannten Methoden gegebenenfalls zu den Sulfoxiden und Sulfonen oxidiert oder

c2) indem man das reaktive Derivat IV, worin $Z_1$ Wasserstoff und $Z_2$ Halogen bedeutet, mit Natriumsulfit umsetzt und die erhaltene Verbindung IV, in welcher $Z_2$ nun $-SO_3H$ bedeutet, in an sich bekannter Weise in die Gruppe $-SO_2NR_4R_5$ umwandelt und im Anschluss an diese gegebenenfalls durchgeführten Umwandlungen der Verbindungen IV die Gruppen W und gegebenenfalls $Z_1$, in die freien Carbonsäuren, ihre Salze, Ester oder Amide überführt, oder

B) eine Verbindung der allgemeinen Formel IV, in welcher $Z_1$ und $Z_2$ gemeinsam eine Oxofunktion darstellen, zunächst mit einem Reduktionsmittel oder einer metallorganischen Verbindung der allgemeinen Formel VII,

$$R_2-M \qquad (VII)$$

in welcher $R_2$ die oben angegebene Bedeutung hat und M ein Alkali- oder Erdalkalimetall bedeutet, umsetzt und die erhaltene Verbindung IV, in welcher $Z_1$ nun einen Rest der Formel $R_2$ und $Z_2$ eine Hydroxylgruppe bedeutet, in an sich bekannter Weise mit einem Sulfonylchlorid der allgemeinen Formel VIII,

$$R_3-SO_2Cl \qquad (VIII)$$

oder einem Carbonsäurechlorid der allgemeinen Formel IX

$$R_3-COCl \qquad (IX)$$

in welchen $R_3$ die oben genannte Bedeutung hat, acyliert und im Anschluss daran gegebenenfalls die Gruppe W in die freie Carbonsäure, ihre Salze, Ester oder Amide überführt.

4. Verbindungen der allgemeinen Formel I'

$$R_1'-A'-\overset{\displaystyle R_2' \atop |}{\underset{| \atop \displaystyle X'}{C}}-COOH \qquad (I')$$

in welcher

$R_1'$ Wasserstoff oder einen gegebenenfalls substituierten Aryl- oder Aryloxyrest,

$R_2'$ Wasserstoff, einen niederen Alkylrest oder die Gruppe $R_1-A'-$, sowie für den Fall, dass X die Cyanogruppe bedeutet, eine Acylamino- oder Aminogruppe,

A' eine geradkettige oder verzweigte, gesättigte oder ungesättigte Alkylengruppe mit 1-18 Kohlenstoffatomen,

X' die Cyano- oder Carbethoxygruppe oder eine Gruppe der Formel $-B-R_3$ oder $-D-NR_4R_5$,
in welcher

B O, S, SO, $SO_2$, O(CO), $OSO_2$, NHCO, $NHSO_2$ oder CO und

D einen Valenzstrich, $SO_2$ oder CO,

$R_3$ eine Alkyl-, Trifluormethyl-, Cycloalkyl-, Aralkyl-, Aralkenyl- oder Arylgruppe, deren Arylrest jeweils substituiert sein kann,

$R_4$ Wasserstoff, einen niederen Alkylrest, einen gegebenenfalls substituierten Aryl- oder Aralkylrest,

$R_5$ Wasserstoff, einen niederen Alkylrest oder

$R_4$ u. $R_5$ gemeinsam eine Alkylenkette mit 4-6 Kohlenstoffatomen, die durch O, S oder $NR_6$ unterbrochen sein kann, und

$R_6$ Wasserstoff, einen niederen Alkylrest oder einen gegebenenfalls substituierten Phenyl- oder Benzylrest

bedeutet, sowie deren physiologisch unbedenklichen Salze, Ester, Amide und Nitrile, zur Verwendung bei der Bekämpfung von Diabetes, Prädiabetes, Adipositas und Atherosklerose.

5. Verbindungen der allgemeinen Formel I

$$R_1-A-\underset{\displaystyle X}{\overset{\displaystyle R_2}{C}}-COOH \qquad (I)$$

in welcher

$R_1$ einen gegebenenfalls substituierten Aryl- oder Aryloxyrest,

$R_2$ Wasserstoff, einen niederen Alkylrest oder die Gruppe $R_1-A-$, sowie für den Fall, dass X die Cyanogruppe bedeutet, eine Acylamino- oder Aminogruppe,

A eine geradkettige oder verzweigte, gesättigte oder ungesättigte Alkylengruppe mit 3-8 Kohlenstoffatomen, die eine Kettenlänge von mindestens 3 Kohlenstoffatomen besitzt,

X die Cyanogruppe oder eine Gruppe der Formel $-B-R_3$ oder $-D-NR_4R_5$,

in welcher

B O, S, SO, $SO_2$, O(CO), $OSO_2$, NHCO, $NHSO_2$ oder CO,

D einen Valenzstrich, $SO_2$ oder CO,

$R_3$ eine Alkyl-, Trifluormethyl-, Cycloalkyl-, Aralkyl-, Aralkenyl- oder Arylgruppe, wobei der jeweilige Arylrest gegebenenfalls substituiert sein kann,

$R_4$ Wasserstoff, einen niederen Alkylrest, einen gegebenenfalls substituierten Aryl- oder Aralkylrest,

$R_5$ Wasserstoff, einen niederen Alkylrest oder

$R_4$ u. $R_5$ gemeinsam eine Alkylenkette mit 4-6 Kohlenstoffatomen, die durch O, S oder $NR_4$ unterbrochen sein kann, und

$R_6$ Wasserstoff, einen niederen Alkylrest oder einen gegebenenfalls substituierten Phenyl- oder Benzylrest

bedeutet, sowie deren physiologisch unbedenklichen Salze, Ester, Amide und Nitrile,

mit der Massgabe, dass für den Fall,

a) dass A eine Alkylengruppe mit 3 Kohlenstoffatomen ist,

$a_1$) in allen Fällen der Arylrest der Gruppe $R_1$ nicht den unsubstituierten Phenylrest und

$a_2$) X nicht die Gruppen $-CN$, $-NHCOR_3$ und $NR_4R_5$,

b) dass X ein Rest der Formel $-SCH_3$ ist, $R_2$ nicht Methyl,

c) dass X die Gruppe $-NH_2$ oder $-NHCOCH_3$ ist,

$R_1A-$ nicht 4-Phenylbutyl oder 4-(4-Methoxyphenyl)butyl,

d) dass X die Gruppe 2,4-Dinitrophenyl ist, $R_1A-$ nicht 4-Phenylbutyl oder 5-Phenylpentyl,

e) dass X die Gruppe $-COCH_3$ ist,

$R_1A$- nicht 3-(2-Chlorphenyl)propyl oder 5-(4-Methoxyphenyl)pentyl

bedeuten darf, zur Verwendung bei der Bekämpfung von Diabetes, Prädiabetes, Adipositas und Atherosklerose.

6. Arzneimittel enthaltend mindestens eine Verbindung der allgemeinen Formel I' gemäss Anspruch 4 sowie übliche Träger- und Hilfsstoffe.

7. Arzneimittel enthaltend mindestens eine Verbindung der allgemeinen Formel I gemäss Anspruch 5 sowie übliche Träger- und Hilfsstoffe.

## Claims

1. Carboxylic acid derivatives of the general formula I

$$R_1-A-\underset{\displaystyle X}{\overset{\displaystyle R_2}{C}}-COOH \qquad (I)$$

in which $R_1$ signifies a possibly substituted aryl or aryloxy radical, $R_2$ hydrogen, a lower alkyl radical or the group $R_1-A-$, as well as for the case that X signifies the cyano group, an acylamino or amino group, A a straight-chained or branched, saturated or unsaturated alkylene group with 3-8 carbon atoms which possesses a chain length of at least 3 carbon atoms, X the cyano group or a group of the formula $-B-R_3$ or $-D-NR_4R_5$, in which B signifies O, S, SO, $SO_2$, O(CO), $OSO_2$, NHCO, $NHSO_2$ or CO, D a valency bond, $SO_2$ or CO, $R_3$ an alkyl, trifluoromethyl, cycloalkyl, aralkyl, aralkenyl or aryl group, whereby the aryl radical in question can possibly be substituted, $R_4$ hydrogen, a lower alkyl radical, a possibly substituted aryl or aralkyl radical, $R_5$ hydrogen, a lower alkyl radical or $R_4$ and $R_5$ together an alkylene chain with 4-6 carbon atoms which can be interrupted by O, S or $NR_6$ and $R_6$ hydrogen, a lower alkyl radical or a possibly substituted phenyl or benzyl radical, as well as their physiologically acceptable salts, esters, amides and nitriles, with the proviso that for the case

a) that A is an alkylene group with 3 carbon atoms,

$a_1$) in all cases, the aryl radical of the group $R_1$ is not the unsubstituted phenyl radical and

$a_2$) X not the groups $-CN$, $-NHCOR_3$ and $-NR_4R_5$,

b) that X is a radical of the formula $-SCH_3$, $R_2$ is not methyl,

c) that X is the group $-NH_2$ or $-NHCOCH_3$, $R_1A-$ is not 4-phenylbutyl or 4-(4-methoxyphenyl)-butyl,

d) that X is the group 2,4-dinitrophenyl, $R_1A-$ is not 4-phenylbutyl or 5-phenylpentyl,

e) that X is the group $-COCH_3$, $R_1A-$ is not 3-(2-chlorophenyl)-propyl or 5-(4-methoxyphenyl)-pentyl.

2. Carboxylic acid derivatives of the general formula I according to claim 1, in which $R_1$ signifies a phenyl, naphthyl, phenyloxy or naphthyloxy radical which can be substituted one or more times by hydroxyl, halogen, alkyl, alkoxy, trifluoromethyl, cyano, nitro, amino, alkylamino and dialkylamino groups, $R_2$ hydrogen, a methyl or ethyl group or the $R_1-A-$, A a straight-chained, saturated or unsaturated alkylene group with 3-8 carbon

atoms, X a cyano, alkoxycarbonyl, alkoxy, alkylsulphenyl, alkylsulphinyl, alkylsulphonyl, trifluoromethylsulphonyl, phenoxy, phenylsulphenyl, phenylsulphinyl, phenylsulphonyl, naphthylsulphonyl, phenylethylenesulphonyl, phenylethenylenesulphonyl, alkylcarbonyloxy, alkylsulphonyloxy, alkylcarbonylamino, alkylsulphonylamino, alkylcarbonyl, phenylcarbonyloxy, phenylsulphonyloxy, phenylcarbonylamino, phenylsulphonylamino, phenylcarbonyl, amino, dialkylamino, phenylamino, benzylamino, morpholino, piperidino, 4-benzylpiperazino, aminosulphonyl, dialkylaminosulphonyl, piperidinosulphonyl, carbamoyl, dialkylaminocarbonyl, phenylaminocarbonyl, benzylaminocarbonyl, piperidinocarbonyl, morpholinocarbonyl, 4-phenylpiperazinocarbonyl and 4-benzylpiperazinocarbonyl, whereby the phenyl ring can in all cases be substituted by the above-mentioned substituents.

3. Process for the preparation of carboxylic acid derivatives of the general formula I

$$R_1-A-\underset{\displaystyle X}{\overset{\displaystyle R_2}{\overset{|}{\underset{|}{C}}}}-COOH \qquad (I)$$

in which $R_1$ signifies a possibly substituted aryl or aryloxy radical, $R_2$ hydrogen, a lower alkyl radical or the group $R_1-A-$, as well as for the case that X signifies the cyano group, an acylamino or amino group, A a straight-chained or branched, saturated or unsaturated alkylene radical with 3-8 carbon atoms which possesses a chain length of at least 3 carbon atoms, X the cyano group or a group of the formula $-B-R_3$ or $-D-NR_4R_5$, in which B is O, SO, $SO_2$, O(CO), $OSO_2$, NHCO, $NHSO_2$ or CO, D a valency bond, $SO_2$ or CO, $R_3$ an alkyl, trifluoromethyl, cycloalkyl, aralkyl, aralkenyl or aryl group, whereby the aryl radical in question can possibly be substituted, $R_4$ hydrogen, a lower alkyl radical, a possibly substituted aryl or aralkyl radical, $R_5$ hydrogen, a lower alkyl radical or $R_4$ and $R_5$ together an alkylene chain with 4-6 carbon atoms which can be interrupted by O, S, $NR_6$ and $R_6$ hydrogen, a lower alkyl radical or a possibly substituted phenyl or benzyl radical, as well as their physiologically acceptable salts, esters, amines and nitriles, with the proviso that for the case

a) that A is an alkylene group with 3 carbon atoms,

$a_1$) in all cases, the aryl radical of the group $R_1$ is not the unsubstituted phenyl radical and

$a_2$) X not the groups $-CN$, $-NHCOR_3$ and $-NR_4R_5$,

b) that X is a radical of the formula $-SCH_3$, $R_2$ is not methyl,

c) that X is the group $-NH_2$ or $NHCOCH_3$, $R_1A-$ is not 4-phenylbutyl or 4-(4-methoxyphenyl)-butyl,

d) that X is the group 2,4-dinitrophenyl, $R_1A-$ is not 4-phenylbutyl or 5-phenylpentyl,

e) that X is the group $-COCH_3$, $R_1A-$, is not 3-(2-chlorophenyl)-propyl or 5-(4-methoxyphenyl)-pentyl,

characterised in that

A) in per se known way, a compound of the general formula II

$$H-\underset{\displaystyle Z_2}{\overset{\displaystyle Z_1}{\overset{|}{\underset{|}{C}}}}-W \qquad (II)$$

in which $Z_1$ signifies $-COOR_7$, $-CN$, $-CO-R_3$, $-SO-R_3$, $-SO_2-R_3$, $-CONH_2$ or $-SO_2NH_2$, $Z_2$ hydrogen, lower alkyl or $-NH-R_8$, W $-COOR_7$ or another convertible into the carboxyl function, $R_7$ lower alkyl and $R_8$ an amino protective group and $R_2$ has the above-given meaning is either

a1) alkylated with a compound of the general formula III

$$R_1-A-Y \qquad (III)$$

in which $R_1$ and A have the above-given meanings and Y represents a reactive residue, or

a2) for the case in which $Z_2$ signifies hydrogen, condensed with a compound of the general formula III'

$$R_1-A'-CHO \qquad (III')$$

in which A' signifies an alkylene radical A shortened by the $-CH_2-$ group and $R_1$ possesses the above-given meaning, and subsequent to the condensation the resultant double bond is hydrogenated and

b) the compounds of general formula IV

$$R_1-A-\underset{\displaystyle Z_2}{\overset{\displaystyle Z_1}{\overset{|}{\underset{|}{C}}}}-W \qquad (IV)$$

obtained from the above-mentioned process steps is possibly converted in that e.g.

b1) for the case that $Z_2$ signifies hydrogen, one again alkylates with a compound of the general formula V

$$R_2-Y \qquad (V)$$

in which $R_2$ possesses the above-given meaning and Y represents a reactive group, or

b2) for the case that $Z_1$ signifies $-COOR_7$ and $Z_2$ the group $-NH-R_8$, converts the radical $R_8$ according to per se known methods into the radical $-CO-R_3$ or $-SO_2-R_3$ or

b3) for the case that $Z_1$ signifies $-COOR_7$ and $Z_2$ hydrogen, exchanges the hydrogen atom in per se known manner by a halogen atom with a halogenation agent, subsequently thereto converts the radical $Z_1$ by decarboxylation into a hydrogen atom and the reactive derivative IV, in which $Z_2$ now stands for halogen and $Z_1$ for hydrogen, is reacted either with a group of the formula VI

$$H-B-R_3 \qquad (VI)$$

in which B stands for O, S or $NHSO_2$ and $R_3$ has

the above-given meaning, to the compound

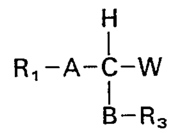

(IV')

or with a group of the general formula VI'

$$HNR_4R_5 \qquad (VI')$$

in which $R_4$ and $R_5$ has the above-given meaning, to the compound

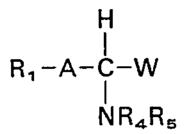

(IV'')

and

c) subsequent to the condensation, for the case that

c1) B signifies a sulphur atom, possibly oxidises according to per se known methods to the sulphoxides or sulphones or

c2) in that one reacts the reactive derivative IV, wherein $Z_1$ signifies hydrogen and $Z_2$ halogen, with sodium sulphite and converts the compound IV obtained, in which $Z_2$ now signifies $-SO_3H$, in per se known manner into the group $-SO_2NR_4R_5$ and, subsequent to these possibly carried out changes of the compounds IV, converts the groups W and possibly $Z_1$ into the free carboxylic acids, their salts, esters or amides, or

B) reacts a compound of general formula IV, in which $Z_1$ and $Z_2$ together represent an oxo function, first with a reducing agent or with an organometallic compound of the general formula VII

$$R_2-M \qquad (VII)$$

in which $R_2$ has the above-given meaning and M signifies an alkali metal or alkaline earth metal, and acylates in per se known manner the compound IV obtained, in which $Z_1$ now signifies a radical of the formula $R_2$ and $Z_2$ a hydroxyl group, with a sulphonyl chloride of the general formula VIII

$$R_3-SO_2Cl \qquad (VIII)$$

or with a carboxylic acid chloride of the general formula IX

$$R_3-COCl \qquad (IX)$$

in which $R_3$ has the above-given meaning, and subsequently possibly converts the group W into the free carboxylic acid, its salts, esters or amides.

4. Compounds of the general formula I'

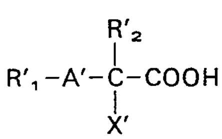

(I')

in which $R'_1$ signifies hydrogen or a possibly substituted aryl or aryloxy radical, $R'_2$ hydrogen, a lower alkyl radical or the group $R_1-A'-$, as well as for the case that X signifies a cyano group, an acylamino or amino group, A' a straight-chained or branched, saturated or unsaturated alkylene group with 1-18 carbon atoms, X' the cyano or carbethoxy group or a group of the formula $-B-R_3$ or $-D-NR_4R_5$, in which B is O, S, SO, $SO_2$, O(CO), $OSO_2$, NHCO, $NHSO_2$ or CO and D a valency bond, $SO_2$ or CO, $R_3$ an alkyl, trifluoromethyl, cycloalkyl, aralkyl, aralkenyl or aryl group, the aryl radical of which can, in each case, be substituted, $R_4$ hydrogen, a lower alkyl radical, a possibly substituted aryl or aralkyl radical, $R_5$ hydrogen, a lower alkyl radical or $R_4$ and $R_5$ together an alkylene chain with 4-6 carbon atoms which can be interrupted by O, S or $NR_6$ and $R_6$ hydrogen, a lower alkyl radical or a possibly substituted phenyl or benzyl radical, as well as their physiologically acceptable salts, esters, amides and nitriles, for use in the combating of diabetes, pre-diabetes, adipositas and atherosclerosis.

5. Compounds of the general formula I

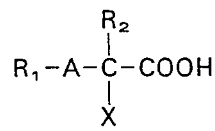

(I)

in which $R_1$ signifies a possibly substituted aryl or aryloxy radical, $R_2$ hydrogen, a lower alkyl radical or the group $R_1-A-$, as well as for the case that X signifies a cyano group, an acylamino or amino group, A a straight-chained or branched, saturated or unsaturated alkylene group with 3-8 carbon atoms which possesses a chain length of at least 3 carbon atoms, X the cyano group or a group of the general formula $-B-R_3$ or $-D-NR_4R_5$, in which B is O, S, SO, $SO_2$, O(CO), $OSO_2$, NHCO, $NHSO_2$ or CO, D a valency bond, $SO_2$ or CO, $R_3$ an alkyl, trifluoromethyl, cycloalkyl, aralkyl, aralkenyl or aryl group, whereby the aryl radicals in question can possibly be substituted, $R_4$ a hydrogen, a lower alkyl radical, a possibly substituted aryl or aralkyl radical, $R_5$ hydrogen, a lower alkyl radical or $R_4$ and $R_5$ together an alkylene chain with 4-6 carbon atoms which can be interrupted by O, S or $NR_6$ and $R_6$ hydrogen, a lower alkyl radical or a possibly substituted phenyl or benzyl radical, as well as their physiologically acceptable salts, esters, amides and nitriles, with the proviso that for the case

a) that A is an alkylene group with 3 carbon atoms,

$a_1$) in all cases, the aryl radical of the group $R_1$ is not the unsubstituted phenyl radical and

$a_2$) X is not the groups $-CN$, $NHCOR_3$ and $-NR_4R_5$,

b) that X is a radical of the formula $-SCH_3$, $R_2$ is not methyl,

c) that X is the group $-NH_2$ or $-NHCOCH_3$, $R_1A-$ is not 4-phenylbutyl or 4-(4-methoxyphenyl)-butyl,

d) that X is the group 2,4-dinitrophenyl, $R_1A-$ is not 4-phenylbutyl or 5-phenylpentyl,

e) that X is the group $-COCH_3$, $R_1A-$ is not 3-(2-chlorophenyl)-propyl or 5-(4-methoxyphenyl)-pentyl,
for use in the combating of diabetes, pre-diabetes, adipositas and atherosclerosis.

6. Medicaments containing at least one compound of the general formula I' according to claim 4, as well as usual carrier and adjuvant materials.

7. Medicaments containing at least one compound of the general formula I acording to claim 5, as well as usual carrier and adjuvant materials.

## Revendications

1. Dérivés d'acide carboxylique de formule générale I

$$R_1-A-\underset{\underset{X}{|}}{\overset{\overset{R_2}{|}}{C}}-COOH \qquad (I)$$

dans laquelle

$R_1$ représente un groupe aryle ou aryloxy éventuellement substitué,

$R_2$ représente un atome d'hydrogène, un groupe alkyle inférieur ou le groupe $R_1-A$, et dans le cas où X représente le groupe cyano, un groupe acylamino ou amino,

A représente un groupe alkylène saturé ou insaturé, à chaîne droite ou ramifiée, comprenant 3-8 atomes de carbone, qui présente une longueur de chaîne d'au moins 3 atomes de carbone,

X un groupe cyano ou un groupe de formule $-B-R_3$ ou $-D-NR_4R_5$, où

B représente O, S, SO, $SO_2$, O(CO), $OSO_2$, NHCO, $NHSO_2$ ou CO,

D une valence libre, $SO_2$ ou CO,

$R_3$ un groupe alkyle, trifluorométhyle, cycloalkyle, aralkyle, aralcényle ou aryle, chaque groupe aryle pouvant être éventuellement substitué,

$R_4$ un atome d'hydrogène, un groupe alkyle inférieur, un groupe alkyle ou aryle éventuellement substitué,

$R_5$ un atome d'hydrogène, un groupe alkyle inférieur ou

$R_4$ et $R_5$ ensemble, une chaîne alkyle avec 4-6 atomes de carbone, qui peut être interrompue par O, S ou $NR_6$, et

$R_6$ un atome d'hydrogène, un groupe alkyle inférieur ou un groupe phényle ou benzyle éventuellement substitué,

ainsi que leurs sels, esters, amides et nitriles physiologiquement acceptables,
avec cette condition que dans le cas où

a) A représente un groupe alkyle avec 3 atomes de carbone,

$a_1$) dans tous les cas, le groupe aryle du groupe $R_1$ n'est pas le groupe phényle non substitué et

$a_2$) X ne représente pas les groupes $-CN$, $-NHCOR_3$ et $NR_4R_5$,

b) X représente un groupe de formule $-SCH_3$, $R_2$ ne représente pas un groupe méthyle,

c) X représente le groupe $-NH_2$ ou $-NHCOCH_3$, $R_1A-$ ne représente pas le groupe 4-phénylbutyle ou 4-(4-méthoxyphényl)butyle,

d) X représente le groupe 2,4-dinitrophényle, $R_1A-$ ne représente pas le groupe 4-phénylbutyle ou 5-phénylpentyle,

e) X représente le groupe $-COCH_3$, $R_1A-$ ne représente pas le groupe 3-(2-chlorophényl)propyle ou 5-(4-méthoxyphényl)pentyle.

2. Dérivés d'acide carboxylique de formule générale selon la revendication 1 où

$R_1$ représente un groupe phényle, naphtyle, phényloxy ou naphtyloxy, qui peut être substitué une ou plusieurs fois par un groupe hydroxyle, un atome d'halogène, un groupe alkyle, alcoxy, trifluorométhyle, cyano, nitro, amino, alkylamino et dialkylamino,

$R_2$ représente un atome d'hydrogène, un groupe méthyle ou éthyle ou le groupe $R_1-A$,

A représente un groupe alkylène saturé ou insaturé, à chaîne droite ou ramifiée, comprenant 3-8 atomes de carbone,

X représente un groupe cyano alcoxycarbonyle, alkylsulfényle, alkylsulfinyle, alkylsulfonyle, trifluorométhylsulfonyle, phénoxy, phénylsulfényle, phénylsulfinyle, phénylsulfonyle, naphtylsulfonyle, phényléthylènesulfonyle, phényléthénylènesulfonyle, alkylcarbonyloxy, alkylsulfonyloxy, alkylcarbonylamino, alkylsulfonylamino, alkylcarbonyl, phénylcarbonyloxy, phénylsulfonyloxy, phénylcarbonylamino, phénylsulfonylamino, phénylcarbonyle, amino, dialkylamino, phénylamino, benzylamino, morpholino, pipéridino, 4-benzylpipérazino, aminosulfonyle, dialkylaminosulfonyle, pipéridinosulfonyle, carbamoyle, dialkylaminocarbonyle, phénylaminocarbonyle, benzylaminocarbonyle, pipéridinocarbonyle, morpholinocarbonyle, 4-phénylpipérazinocarbonyle et 4-benzylpipérazinocarbonyle, le noyau phényle pouvant être substitué dans tous les cas par les substituants mentionnés ci-dessus.

3. Procédé pour la préparation de dérivés d'acide *carboxylé* de formule générale I

$$R_1-A-\underset{\underset{X}{|}}{\overset{\overset{R_2}{|}}{C}}-COOH \qquad (I)$$

dans laquelle

$R_1$ représente un groupe aryle ou aryloxy éventuellement substitué,

$R_2$ représente un atome d'hydrogène, un groupe alkyle inférieur ou le groupe $R_1-A$, et dans le cas où X représente le groupe cyano, un groupe acylamino ou amino,

A représente un groupe alkylène saturé ou insaturé, à chaîne droite ou ramifiée, comprenant 3-8 atomes de carbone, qui présente une longueur de chaîne d'au moins 3 atomes de carbone,

X un groupe cyano ou un groupe de formule $-B-R_3$ ou $-D-NR_4R_5$, où

B représente O, S, SO, $SO_2$, O(CO), $OSO_2$, NHCO, $MHSO_2$ ou CO,

D une valence libre, $SO_2$ ou CO,

$R_3$ un groupe alkyle, trifluorométhyle, cycloalkyle, aralkyle, aralcényle ou aryle, chaque groupe aryle pouvant être éventuellement substitué,

$R_4$ un atome d'hydrogène, un groupe alkyle inférieur, un groupe alkyle ou aryle éventuellement substitué,

$R_5$ un atome d'hydrogène, un groupe alkyle inférieur ou

$R_4$ et $R_5$ ensemble, une chaîne alkyle avec 4-6 atomes de carbone, qui peut être interrompue par O, S ou $NR_6$, et

$R_6$ un atome d'hydrogène, un groupe alkyle inférieur ou un groupe phényle ou benzyle éventuellement substitué,

ainsi que leurs sels, esters, amides et nitriles physiologiquement acceptables,

avec cette condition que dans le cas où

a) A représente un groupe alkyle avec 3 atomes de carbone,

$a_1$) dans tous les cas, le groupe aryle du groupe $R_1$ n'est pas le groupe phényle non substitué, et

$a_2$) X ne représente pas les groupes $-CN$, $-NHCOR_3$ et $NR_4R_5$,

b) X représente un groupe de formule $-SCH_3$, $R_2$ ne représente pas un groupe méthyle,

c) X représente le groupe $-NH_2$ ou $-NHCOCH_3$, $R_1A-$ ne représente pas le groupe 4-phénylbutyle ou 4-(4-méthoxyphényl)butyle,

d) X représente le groupe 2,4-dinitrophényle, $R_1A-$ ne représente pas le groupe 4-phénylbutyle ou 5-phénylpentyle,

e) X représente le groupe $-COCH_3$, $R_1A-$ ne représente pas le groupe 3-(2-chlorophényl)propyle ou 5-(4-méthoxyphényl)pentyle,

caractérisé en ce que

A) d'une manière connue en soi, on fait réagir un composé de formule générale II

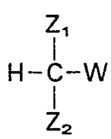 (II)

où les symboles représentent:

$Z_1$ $-COOR_7$, $-CN$, $-CO-R_3$, $-SO-R_3$, $-SO_2-R_3$, $-CONH_2$ ou $-SO_2NH_2$,

$Z_2$ un atome d'hydrogène, un groupe alkyle inférieur ou $-NH-R_9$

W $-COOR_7$ ou un autre groupe transformable en fonction carbonyle,

$R_7$ un groupe alkyle inférieur et

$R_8$ un groupe de protection amino et $R_2$ a la signification mentionnée plus haut, soit

a1) par alkylation avec un composé de formule générale III,

$$R_1-A-Y \qquad (III)$$

où $R_1$ et A ont la signification mentionnée plus haut, et Y représente un groupe réactif, soit

a2) dans le cas où $Z_2$ représente un atome d'hydrogène, par condensation avec un composé de formule générale III'

$$R_1-A'-CHO \qquad (III')$$

où A' représente un groupe alkylène réduit d'un groupe $CH_2$, et R a la signification mentionnée plus haut, et, à la suite de la condensation, on hydrogène la double liaison formée et

b) on transforme éventuellement le composé de formule générale IV obtenu dans les étapes de procédé ci-dessus

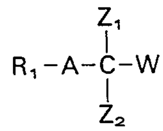 (IV)

en ce que par exemple

b1) dans le cas où $Z_2$ représente un atome d'hydrogène, on effectue de nouveau une alkylation avec un composé de formule générale V

$$R_2-Y \qquad (V)$$

où $R_2$ a la signification mentionnée ci-dessus et Y représente un groupe réactif, ou bien

b2) dans le cas où $Z_1$ représente $-COOR_7$ et $Z_2$ le groupe $-NH-R_8$, on transforme le groupe $R_8$, selon des méthodes connues en soi, en groupes $-CO-R_3$ ou $-SO_2R_3$, ou

b3) dans le cas où $Z_1$ représente $-COOR_7$ et $Z_2$ un atome d'hydrogène, on échange l'atome d'hydrogène de manière connue en soi, à l'aide d'un agent d'halogénation, contre un atome d'halogène, puis on transforme le groupe $Z_1$ par décarboxylation en un atome d'hydrogène, et on fait réagir le dérivé réactif IV, dans lequel maintenant $Z_2$ représente un atome d'halogène et $Z_1$ un atome d'hydrogène, soit avec un groupe de formule VI,

$$H-B-R_3 \qquad (VI)$$

dans lequel B représente O, S ou $NHSO_2$ et $R_3$ a la signification indiquée ci-dessus, de façon à obtenir le composé

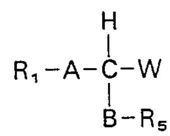 (IV')

ou avec un groupe de formule VI',

$$HNR_4R_5 \qquad (VI')$$

où $R_4$ et $R_5$ ont la signification mentionnée ci-dessus, pour obtenir le composé

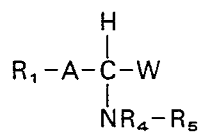 (IV")

et

c) après la condensation, dans le cas où

c1) B représente un atome de soufre, on oxyde éventuellement en sulfoxyde ou sulfone, selon les méthodes connues, ou

c2) on fait réagir le dérivé IV réactif, dans lequel $Z_1$ représente un atome d'hydrogène et $Z_2$ un atome d'halogène, avec le sulfite de sodium et on transforme le composé IV obtenu, dans lequel $Z_2$ représente désormais $-SO_3H$, de manière connue en soi, en groupe $-SO_2NR_4R_5$ et après ces transformations effectuées éventuellement sur le composé IV, on transforme les groupes W et éventuellement $Z_1$, en acides carboxyliques libres, en leurs sels, leurs esters ou leurs amides, ou

B) on fait réagir un composé de formule générale IV, dans lequel $Z_1$ et $Z_2$ représentent ensemble une fonction oxo, d'abord avec un agent de réduction ou un composé organométallique de formule générale VII,

$$R_2-M \qquad (VII)$$

où $R_2$ a la signification mentionnée ci-dessus et M représente un métal alcalin ou alcalino-terreux, et on effectue l'acylation du composé IV obtenu, dans lequel désormais $Z_1$ représente un groupe de formule $R_2$ et $Z_2$ un groupe hydroxyle, de manière connue en soi, avec un chlorure de sulfonyle de formule générale VIII,

$$R_3-SO_2Cl \qquad (VIII)$$

ou avec un chlorure d'acide carboxylique de formule générale IX

$$R_3-COCl \qquad (IX)$$

où $R_3$ a la signification mentionnée plus haut, et ensuite, on transforme éventuellement le groupe W en acide carboxylique libre, en son sel, ester ou amide.

4. Composés de formule générale I'

$$\begin{array}{l} \qquad\qquad R_2' \\ \qquad\qquad | \\ R_1'-A'-C-COOH \\ \qquad\qquad | \\ \qquad\qquad X' \end{array} \qquad (I')$$

dans laquelle

$R_1'$ représente un atome d'hydrogène ou un groupe aryle ou aryloxy éventuellement substitué,

$R_2'$ représente un atome d'hydrogène, un groupe alkyle inférieur ou le groupe $R_1-A'$, et dans le cas où X représente le groupe cyano, un groupe acylamino ou amino,

A' représente un groupe alkylène saturé ou insaturé, à chaîne droite ou ramifiée, comprenant 1-18 atomes de carbone,

X' un groupe cyano ou un groupe de formule $-B-R_3$ ou $-D-NR_4R_5$, où

B représente O, S, SO, $SO_2$, O(CO), $OSO_2$, NHCO, $NHSO_2$ ou CO, et

D une valence libre, $SO_2$ ou CO,

$R_3$ un groupe alkyle, trifluorométhyle, cycloalkyle, aralkyle, aralcényle ou aryle, chaque groupe aryle pouvant être éventuellement substitué,

$R_4$ un atome d'hydrogène, un groupe alkyle inférieur, un groupe alkyle ou aryle éventuellement substitué,

$R_5$ un atome d'hydrogène, un groupe alkyle inférieur ou

$R_4$ et $R_5$ ensemble, une chaîne alkyle avec 4-6 atomes de carbone, qui peut être interrompue par O, S ou $NR_5$, et

$R_6$ un atome d'hydrogène, un groupe alkyle inférieur ou un groupe phényle ou benzyle éventuellement substitué,

ainsi que leurs sels, esters, amides et nitriles physiologiquement acceptables, pour l'utilisation dans la lutte contre le diabète, le prédiabète, l'adiposité et l'athérosclérose.

5. Composés de formule générale I

$$\begin{array}{l} \qquad\qquad R_2 \\ \qquad\qquad | \\ R_1-A-C-COOH \\ \qquad\qquad | \\ \qquad\qquad X \end{array} \qquad (I)$$

dans laquelle

$R_1$ représente un groupe aryle ou aryloxy éventuellement substitué,

$R_2$ représente un atome d'hydrogène, un groupe alkyle inférieur ou le groupe $R_1-A$, et dans le cas ou X représente le groupe cyano, un groupe acylamino ou amino,

A représente un groupe alkylène saturé ou insaturé, à chaîne droite ou ramifiée, comprenant 3-8 atomes de carbone, qui présente une longueur de chaîne d'au moins 3 atomes de carbone,

X un groupe cyano ou un groupe de formule $-B-R_3$ ou $-D-NR_4R_5$, où

B représente O, S, SO, $SO_2$, O(CO), $OSO_2$, NHCO, $NHSO_2$ ou CO,

D une valence libre, $SO_2$ ou CO,

$R_3$ un groupe alkyle, trifluorométhyle, cycloalkyle, aralkyle, aralcényle ou aryle, chaque groupe aryle pouvant être éventuellement substitué,

$R_4$ un atome d'hydrogène, un groupe alkyle inférieur, un groupe alkyle ou aryle éventuellement substitué,

$R_5$ un atome d'hydrogène, un groupe alkyle inférieur ou

$R_4$ et $R_5$ ensemble, une chaîne alkyle 4-6 atomes de carbone, qui peut être interrompue par O, S ou $NR_6$, et

$R_6$ un atome d'hydrogène, un groupe alkyle inférieur ou un groupe phényle ou benzyle éventuellement substitué,

ainsi que leurs sels, esters, amides et nitriles physiologiquement acceptables, avec cette condition que dans le cas où

a) A représente un groupe alkyle avec 3 atomes de carbone,

$a_1$) dans tous les cas, le groupe aryle du groupe $R_1$ n'est pas le groupe phényle non substitué, et

$a_2$) X ne représente pas les groupes $-CN$, $-NHCOR_3$ et $NR_4R_5$,

b) X représente un groupe de formule $-SCH_3$, $R_2$ ne représente pas un groupe méthyle,

c) X représente le groupe $-NH_2$ ou $-NHCOCH_3$, $R_1A-$ ne représente pas le groupe 4-phénylbutyle ou 4-(4-méthoxyphényl)butyle,

d) X représente le groupe 2,4-dinitrophényle, $R_1A-$ ne représente pas le groupe 4-phénylbutyle ou 5-phénylpentyle,

e) X représente le groupe $-COCH_3$, $R_1A-$ ne représente pas le groupe 3-(2-chlorophényl)propyle ou 5-(4-méthoxyphényl)pentyle, destinés à l'utilisation dans la lutte contre le diabète, le prédiabète, l'adiposité et l'athérosclérose.

6. Médicament contenant au moins un composé de formule générale I' selon la revendication 4, ainsi que des matières de support ou auxiliaires habituelles.

7. Médicament contenant au moins un composé de formule générale I selon la revendication 5, ainsi que des matières de support ou auxiliaires habituelles.